# EUROPEAN PATENT SPECIFICATION

(11) **EP 3 521 409 B1**
(45) Date of publication and mention of the grant of the patent: **14.08.2024**
(21) Application number: 19164800.5
(22) Date of filing: 26.03.2015
(51) Int. Cl.: C11B 9/00, A23L 27/20, C07C 33/025

(54) **PERFUME SYSTEMS**
PARFÜMSYSTEME
SYSTÈMES DE PARFUM

(30) Priority: 26.03.2014 US 201461970385 P
(43) Date of publication of application: 07.08.2019
(62) Divisional of application: 15715933.6
(73) Proprietor: The Procter & Gamble Company, Cincinnati, OH 45202 (US)
(72) Inventor: DENUTTE, Hugo Robert Germain, 1853 Strombeek-Bever (BE); PINTENS, An, 1853 Strombeek-Bever (BE); SMETS, Johan, 1853 Strombeek-Bever (BE); VRIELYNCK, Freek Annie Camiel, 8400 Ooostende (BE); VAN AKEN, Koen, 8400 Ooostende (BE)
(74) Representative: P&G Patent Belgium UK

(56) References cited:
- EP-A1- 0 761 629
- EP-A1- 1 029 841
- EP-A1- 1 837 326
- EP-A2- 1 066 824
- WO-A1-2010/052635
- CN-A- 102 617 340
- GB-A- 1 270 412
- GB-A- 786 349
- US-A- 3 452 105
- US-A- 4 482 762
- US-A- 4 572 795
- US-A- 4 710 316
- US-A- 5 358 930
- US-A1- 2013 035 278
- CHEVES WALLING AND HARVEY J. SCHUGAR: "Organic Reactions under High Pressure. VII. Volumes of Activation for Some Diels-Alder Reactions", JOURNAL OF THE AMERICAN CHEMICAL SOCIETY, vol. 85, 5 March 1963 (1963-03-05), pages 607 - 612, XP002793768
- GRAMENITSKAYA V. N. ET AL.: "Alternative participation of the multiple bonds of alpha,beta-unsaturated aldehydes in the diene synthesis reaction", JOURNAL OF ORGANIC CHEMISTRY OF THE USSR, vol. 13, 1977, pages 2169 - 2175, XP009515476
- JACK E. BALDWIN AND MICHAEL J. LUSCH: "Rearrangements in Lewis Acid Catalyzed Diels-Alder Reactions. Route to Substituted Bicyclo[2.2.1]heptanones", JOURNAL OF ORGANIC CHEMISTRY, vol. 44, no. 12, 1979, pages 1923 - 1927, XP002793769
- BAH JUHO ET AL: "Carbocations as Lewis acid catalysts in Diels-Alder and Michael addition reactions.", CHEMISTRY (WEINHEIM AN DER BERGSTRASSE, GERMANY) 20 JAN 2014, vol. 20, no. 4, 20 January 2014 (2014-01-20), pages 1066 - 1072, XP002793770, ISSN: 1521-3765
- ESBEN TAARNING ET AL: "Unsaturated Aldehydes as Alkene Equivalents in the Diels-Alder Reaction", CHEMISTRY - A EUROPEAN JOURNAL, vol. 14, no. 18, 9 May 2008 (2008-05-09), DE, pages 5638 - 5644, XP055315232, ISSN: 0947-6539, DOI: 10.1002/chem.200800003

## Description

### FIELD OF INVENTION

The present application relates to 6-(*R*,*S*)-ethyl-6,9-dimethyldec-8-en-5-ol and stereoisomers thereof as perfume raw materials, perfume delivery systems comprising them, and consumer products comprising such perfume raw materials and/or perfume delivery systems.

### BACKGROUND OF THE INVENTION

Consumer products may comprise one or more perfumes, and/or perfume delivery systems that can mask an undesirable odor and/or provide a desired scent and/or experience to a product and/or a situs that is contacted with such a product. While current perfumes, and perfume delivery systems provide desirable experiences and/or fragrances, consumers continue to seek products that contain sensates, such as cooling or have scents that may be longer lasting and that are tailored to their individual desires (see for example USPA 2007/0275866 A1 and USPA 2008/0305977 A1) - unfortunately the pool of perfume raw materials and perfume delivery systems that is available is still too limited to completely meet the desired needs. EP 1837326 A1 is directed to novel organoleptic compounds and their use in perfume compositions. EP 1029841 A1 is concerned with mixtures of the (6E)- and (6Z)-isomers of 3,6-dimethyloct-6-en-1-ols and 6-ethyl-3-methyloct-6-en-1-ols and their mixtures as well as the use of these mixtures in odorant compositions. The mixtures are free from their corresponding oct-5-ene double bond isomers. US 4572795 A is concerned with compounds of the formula: I 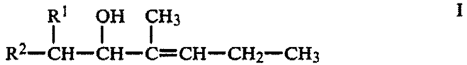 wherein: R1 is selected from the group consisting of hydrogen and methyl; R2 is selected from the group consisting of normal or terminally singly branched alkyl or alkenyl groups having four to seven carbon atoms provided that: (i) when R1 is hydrogen the alkyl group must have at least five carbon atoms, and (ii) when R1 is methyl, R2 is an alkyl group. This invention is also concerned with odorant compositions containing these compounds.

US 4482762 A is concerned with compounds of the formula: I 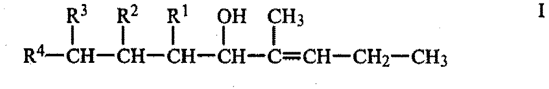 wherein one of the symbols R1, R2 and R3 stands for methyl or ethyl and the others stand for hydrogen and R4 signifies hydrogen or methyl, with the proviso that R4 represents hydrogen when R1 and R2 both represent hydrogen and R3 represents methyl and their use as odorants and flavorants.

GB 786 349 A relates dienic tertiary alcohols of specific formula and the corresponding mono-olefinic alcohols The products may be used in the preparation of oderiferous compositions and perfumes. EP 0761629 A1 relates to open chain branched olefinically unsaturated alcohols and esters of formula CH3-C(CH3)=CH-CH2-C(CH3)2-CR1R2R3 (I). R1 = OH, O-CHO or -O-CO-A; A = 1-6 C alkyl; R2 = H or 1-6 C alkyl; and R3 = H, 1-4 C alkyl or vinyl; provided that R2 and R3 are not both = H when R1 = OH. US 3452105 A is directed to 3-hydroxy-7-isobutyl-1,6-octadiene, which is useful as an odorant in perfumes and other scented compositions.

Applicants believe that the perfume raw materials and perfumes, including the delivery systems, disclosed herein expand the options, as such sensates and/or perfume raw materials can provide variations on character and such and/or perfumes can provide desired sensations and/or odor profiles. In certain aspects, such and/or perfume raw materials and/or perfume delivery systems comprising such and/or perfume raw materials may provide variations on character, sensation and/or odor profiles that are better than expected as measured by parameters such as headspace analysis (employed to determine perfume delivery system perfume leakage and/or perfume delivery efficiency), ClogP, boiling point and/or odor detection threshold.

### SUMMARY OF THE INVENTION

The invention is set out in the appended claims.

### DETAILED DESCRIPTION OF THE INVENTION

### Definitions

As used herein "consumer product" means baby care, beauty care, fabric & home care, family care, feminine care, health care, snack and/or beverage products or devices generally intended to be used or consumed in the form in which it is sold. Such products include but are not limited to diapers, bibs, wipes; products for and/or methods relating to treating hair (human, dog, and/or cat), including, bleaching, coloring, dyeing, conditioning, shampooing, styling; deodorants and antiperspirants; personal cleansing; cosmetics; skin care including application of creams, lotions, and other topically applied products for consumer use including fine fragrances; and shaving products, products for and/or methods relating to treating fabrics, hard surfaces and any other surfaces in the area of fabric and home care, including: air care including air fresheners and scent delivery systems, car care, dishwashing, fabric conditioning (including softening and/or freshening), laundry detergency, laundry and rinse additive and/or care, hard surface cleaning and/or treatment including floor and toilet bowl cleaners, and other cleaning for consumer or institutional use; products and/or methods relating to bath tissue, facial tissue, paper handkerchiefs, and/or paper towels; tampons, feminine napkins; products and/or methods relating to oral care including toothpastes, tooth gels, tooth rinses, denture adhesives, tooth whitening; over-the-counter health care including cough and cold remedies, pain relievers, RX pharmaceuticals, pet health and nutrition; processed food products intended primarily for consumption between customary meals or as a meal accompaniment (non-limiting examples include potato chips, tortilla chips, popcorn, pretzels, corn chips, cereal bars, vegetable chips or crisps, snack mixes, party mixes, multigrain chips, snack crackers, cheese snacks, pork rinds, corn snacks, pellet snacks, extruded snacks and bagel chips); and coffee.

As used herein, the term "cleaning and/or treatment composition" is a subset of consumer products that includes, unless otherwise indicated, beauty care, fabric & home care products. Such products include, but are not limited to, products for treating hair (human, dog, and/or cat), including, bleaching, coloring, dyeing, conditioning, shampooing, styling; deodorants and antiperspirants; personal cleansing; cosmetics; skin care including application of creams, lotions, and other topically applied products for consumer use including fine fragrances; and shaving products, products for treating fabrics, hard surfaces and any other surfaces in the area of fabric and home care, including: air care including air fresheners and scent delivery systems, car care, dishwashing, fabric conditioning (including softening and/or freshening), laundry detergency, laundry and rinse additive and/or care, hard surface cleaning and/or treatment including floor and toilet bowl cleaners, granular or powder-form all-purpose or "heavy-duty" washing agents, especially cleaning detergents; liquid, gel or paste-form all-purpose washing agents, especially the so-called heavy-duty liquid types; liquid fine-fabric detergents; hand dishwashing agents or light duty dishwashing agents, especially those of the high-foaming type; machine dishwashing agents, including the various tablet, granular, liquid and rinse-aid types for household and institutional use; liquid cleaning and disinfecting agents, including antibacterial hand-wash types, cleaning bars, mouthwashes, denture cleaners, dentifrice, car or carpet shampoos, bathroom cleaners including toilet bowl cleaners; hair shampoos and hair-rinses; shower gels , fine fragrances and foam baths and metal cleaners; as well as cleaning auxiliaries such as bleach additives and "stain-stick" or pre-treat types, substrate-laden products such as dryer added sheets, dry and wetted wipes and pads, nonwoven substrates, and sponges; as well as sprays and mists all for consumer or/and institutional use; and/or methods relating to oral care including toothpastes, tooth gels, tooth rinses, denture adhesives, tooth whitening.

As used herein, the term "fabric and/or hard surface cleaning and/or treatment composition" is a subset of cleaning and treatment compositions that includes, unless otherwise indicated, granular or powder-form all-purpose or "heavy-duty" washing agents, especially cleaning detergents; liquid, gel or paste-form all-purpose washing agents, especially the so-called heavy-duty liquid types; liquid fine-fabric detergents; hand dishwashing agents or light duty dishwashing agents, especially those of the high-foaming type; machine dishwashing agents, including the various tablet, granular, liquid and rinse-aid types for household and institutional use; liquid cleaning and disinfecting agents, including antibacterial hand-wash types, cleaning bars, car or carpet shampoos, bathroom cleaners including toilet bowl cleaners; and metal cleaners, fabric conditioning products including softening and/or freshening that may be in liquid, solid and/or dryer sheet form ; as well as cleaning auxiliaries such as bleach additives and "stain-stick" or pre-treat types, substrate-laden products such as dryer added sheets, dry and wetted wipes and pads, nonwoven substrates, and sponges; as well as sprays and mists. All of such products which were applicable may be in standard, concentrated or even highly concentrated form even to the extent that such products may in certain aspect be non-aqueous.

As used herein, the term "oral care composition" is a product, which in the ordinary course of usage, is not intentionally swallowed for purposes of systemic administration of particular therapeutic agents, but is rather retained in the oral cavity for a time sufficient to contact substantially all of the dental surfaces and/or oral tissues for purposes of oral activity. The oral care composition may be in various forms including toothpaste, dentifrice, tooth gel, subgingival gel, mouthrinse, mousse, foam, mouthspray, lozenge, chewable tablet, chewing gum or denture product. The oral care composition may also be incorporated onto strips or films for direct application or attachment to oral surfaces. The term "dentifrice", as used herein, includes paste, gel, or liquid formulations unless otherwise specified.
The dentifrice composition may be a single phase composition or may be a combination of two or more separate dentifrice compositions. The dentifrice composition may be in any desired form, such as deep striped, surface striped, multilayered, having a gel surrounding a paste, or any combination thereof. Each dentifrice composition in a dentifrice comprising two or more separate dentifrice compositions may be contained in a physically separated compartment of a dispenser and dispensed side-by-side.

As used herein, articles such as "a" and "an" when used in a claim, are understood to mean one or more of what is claimed or described.

As used herein, the terms "include", "includes" and "including" are meant to be non-limiting.

As used herein, the term "solid" includes granular, powder, bar and tablet product forms.

As used herein, the term "fluid" includes liquid, gel, paste and gas product forms.

As used herein, the term "situs" includes paper products, fabrics, garments, hard surfaces, hair and skin.

As used herein, "perfume raw materials" include molecules that can serve the purposes of providing odour and/or a sensation such as cooling.

Unless otherwise noted, all component or composition levels are in reference to the active portion of that component or composition, and are exclusive of impurities, for example, residual solvents or by-products, which may be present in commercially available sources of such components or compositions.

All percentages and ratios are calculated by weight unless otherwise indicated. All percentages and ratios are calculated based on the total composition unless otherwise indicated.

It should be understood that every maximum numerical limitation given throughout this specification includes every lower numerical limitation, as if such lower numerical limitations were expressly written herein. Every minimum numerical limitation given throughout this specification will include every higher numerical limitation, as if such higher numerical limitations were expressly written herein. Every numerical range given throughout this specification will include every narrower numerical range that falls within such broader numerical range, as if such narrower numerical ranges were all expressly written herein.

**Table 1 Molecules (also known as "PRMs")**

| Molecule Number 20 listed in Table 1 below and stereoisomers thereof are according to the invention; the other Molecules listed in Table 1 below are not according to the invention. | | | |
|---|---|---|---|
| Number | Chemical Structure | IUPAC name | Character description |
| **1** | 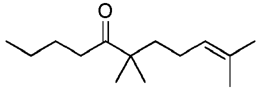 | 6,6,10-trimethylundec-9-en-5-one | green (geranium), vegetable, violet |
| **2** | 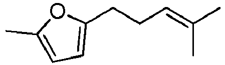 | 2-methyl-5-(4-methylpent-3-en-1-yl)furan | herbal, anisic, floral |
| **3** | 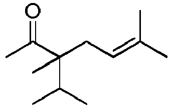 | 3-isopropyl-3,6-dimethylhept-5-en-2-one | herbal, aromatic |
| **4** | 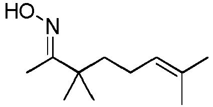 | (*E*,*Z*)-3,3,7-trimethyloct-6-en-2-one oxime | green, earthy |
| **5** | 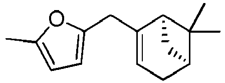 | 2-(((1*R*,5*S*)-6,6-dimethylbicyclo[ 3.1.1]hept-2-en-2-yl)methyl)-5-methylfuran | woody, terpenic |
| **6** | 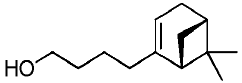 | 4-((IR,5S)-6,6-dimethylbicyclo[ 3.1.1]hept-2-en-2-yl)butan-1-ol | woody, earthy |
| **7** | 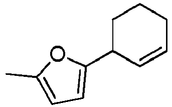 | 2-(cyclohex-2-en-1-(*R*,*S*)-yl)-5-methylfuran | green (chervil), herbal |
| **8** | 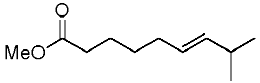 | methyl 8-methylnon-6-enoate | fruity |
| **9** | 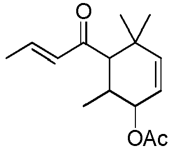 | 5-(but-2-enoyl)-4,4,6-trimethylcyclohe x-2-en-1-yl acetate | apple - rose ketone smell |
| **10** | 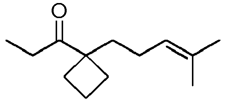 | 1-(1-(4-methylpent-3-en-1-yl)cyclobutyl)pr opan-1-one | fruity (pineapple, currant - grenadine), minty |
| **11** | 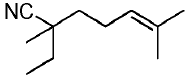 | 2-(*R*,*S*)-ethyl-2,6-dimethylhept-5-enenitrile | herbal (fennel), fruity |
| **12** | 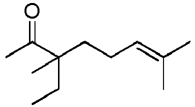 | 3-(*R*,*S*)-ethyl-3,7-dimethyloct-6-en-2-one | woody, herbal (lavender) |
| **13** | 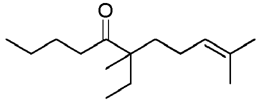 | 6-(*R*,*S*)-ethyl-6,10-dimethylundec-9-en-5-one | herbal, floral |
| **14** | 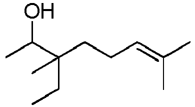 | 3-(*R*,*S*)-ethyl-3,7-dimethyloct-6-en-2-(*R*,*S*)-ol | floral (lilac), clean |
| **15** | 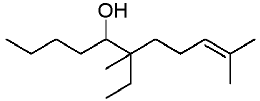 | 6-(*R*,*S*)-ethyl-6,10-dimethylundec-9-en-5-(*R*,*S*)-ol | floral, herbal, |
| **16** | 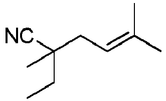 | 2-(*R*,*S*)-ethyl-2,5-dimethylhex-4-enenitrile | herbal, minty (carway), spicy (celery) |
| **17** | 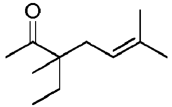 | 3-(*R*,*S*)-ethyl-3,6-dimethylhept-5-en-2-one | citrus, fruity, fresh |
| **18** | 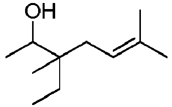 | 3-(*R*,*S*)-ethyl-3,6-dimethylhept-5-en-2-ol | floral, herbal (lilac, pine), clean |
| **19** | 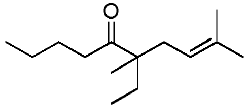 | 6-(*R*,*S*)-ethyl-6,9-dimethyldec-8-en-5-one | woody, leathery |
| **20** | 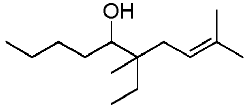 | 6-(*R*,*S*)-ethyl-6,9-dimethyldec-8-en-5-ol | floral (lily of the valley) |
| **21** | 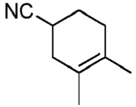 | (*R*,*S*)-3,4-dimethylcyclohe x-3-enecarbonitrile | almond |
| **22** | 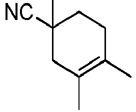 | 1-(*R*,*S*)-,3,4-trimethylcyclohe x-3-enecarbonitrile | herbal, spicy (nutty) |
| **23** | 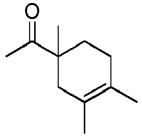 | 1-(1-(*R*,*S*),3,4-trimethylcyclohe x-3-en-1-yl)ethanone | fresh, citrus, herbal, woody |
| **24** | 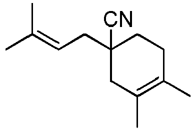 | 3,4-dimethyl-1-(*R*,*S*)-(3-methylbut-2-en-1-yl)cyclohex-3-enecarbonitrile | mussel, terpenic |
| **25** | 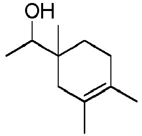 | 1-(*R*,*S*)-(1-(*R*,*S*),3,4-trimethyl-cyclohex-3-en-1-yl)ethanol | clean, pine, floral (lily of the valley) |
| **26** | 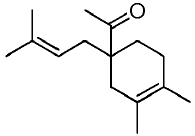 | 1-(3,4-dimethyl-1-(*R*,*S*)-(3-methylbut-2-en-1-yl)cyclohex-3-en-1-yl)ethanone | clean, floral, woody (pine), green |
| **27** | 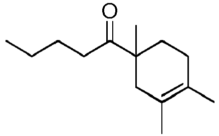 | 1-(1-(*R*,*S*),3,4-trimethylcyclohe x-3-en-1-yl)pentan-1-one | woody, spicy |
| **28** | 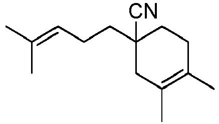 | 3,4-dimethyl-1-(*R*,*S*)-(4-methylpent-3-en-1-yl)cyclohex-3-enecarbonitrile | green |
| **29** | 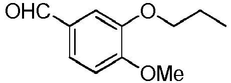 | 4-methoxy-3-propoxybenzalde hyde | vanilla, caramel |
| **30** | 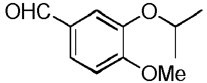 | 3-isopropoxy-4-methoxybenzald ehyde | nutty, roasted |
| **31** | 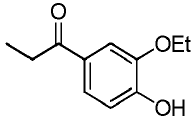 | 1-(3-ethoxy-4-hydroxyphenyl)p ropan-1-one | sweet vanilla, heliotropine |
| **32** | 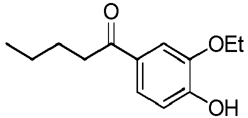 | 1-(3-ethoxy-4-hydroxyphenyl)p entan-1-one | sweet powder |
| **33** | 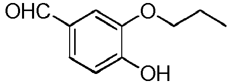 | 4-hydroxy-3-propoxybenzalde hyde | sweet, medicinal, mint |
| **34** | 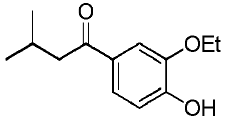 | 1-(3-ethoxy-4-hydroxyphenyl)-3-methylbutan-1-one | sweet balsamic, buttery |
| **35** | 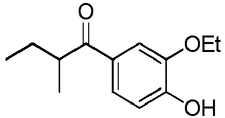 | 1-(3-ethoxy-4-hydroxyphenyl)-2-methylbutan-1-one | sweet, almond, green |
| **36** | 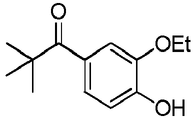 | 1-(3-ethoxy-4-hydroxyphenyl)-2,2-dimethylpropan-1-one | sweet, dusty |
| **37** | 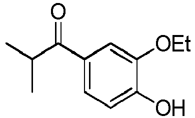 | 1-(3-ethoxy-4-hydroxyphenyl)-2-methylpropan-1-one | sweet, phenolic, woody |
| **38** | 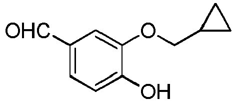 | 3-(cyclopropylmet hoxy)-4-hydroxybenzalde hyde | sweet, floral, anisic |
| **39** | 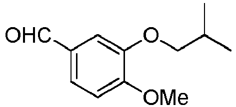 | 3-isobutoxy-4-methoxybenzald ehyde | sweet |
| 40 | 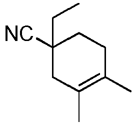 | 1-(*R*,*S*)-ethyl-3,4-dimethyl-cyclohex-3-enecarbonitrile | spicy |
| **41** | 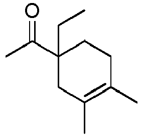 | 1-(1-(*R*,*S*)-ethyl-3,4-dimethyl-cyclohex-3-en-1-yl)ethanone | floral, pine needles, fresh (lime) |
| **42** | 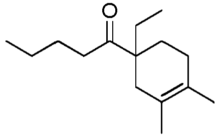 | 1-(1-(*R*,*S*)-ethyl-3,4-dimethyl-cyclohex-3-en-1-yl)pentan-1-one | chewing gum (fruity) |
| **43** | 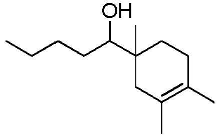 | 1-(1-(*R*,*S*)-methyl-3,4-dimethyl-cyclohex-3-en-1-yl)pentan-1-ol | fruity, herbal, chamomile |
| **44** | 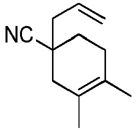 | 1-(*R*,*S*)allyl-3,4-dimethylcyclohe x-3-enecarbonitrile | green, violet |
| **45** | 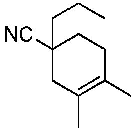 | 3,4-dimethyl-1-(*R*,*S*)-propylcyclohex-3-enecarbonitrile | citrus, herbal |
| **46** | 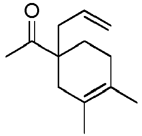 | 1-(1-(*R*,*S*)-allyl-3,4-dimethyl-cyclohex-3-en-1-yl)ethanone | herbal (lavender), citrus, sweet |
| **47** | 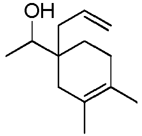 | 1-(1-(*R*,*S*)-allyl-3,4-dimethyl-cyclohex-3-en-1-yl)ethanol | woody |
| **48** | 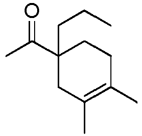 | 1-(3,4-dimethyl-1-(*R*,*S*)-propyl-cyclohex-3-en-1-yl)ethanone | fresh, citrus, floral, bergamot |
| **49** | 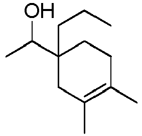 | 1-(3,4-dimethyl-1-(*R*,*S*)propyl-cyclohex-3-en-1-yl)ethanol | hay, carrotseed |
| **50** | 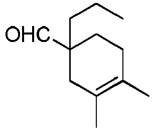 | 3,4-dimethyl-1-(*R*,*S*)-propyl-cyclohex-3-enecarbaldehyde | citrus, aldehydic |
| **51** | 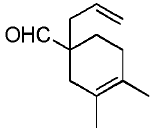 | 1-(*R*,*S*)-allyl-3,4-dimethyl-cyclohex-3-enecarbaldehyde | mint, herbal, tea, violet |
| **52** | 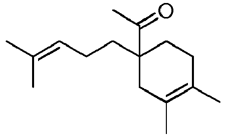 | 1-(*R*,*S*)-(3,4-dimethyl-1-(4-methylpent-3-enyl)cyclohex-3-enyl)ethanone | floral, green |
| **53** | 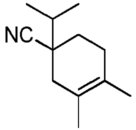 | 1-(*R*,*S*)-isopropyl-3,4-dimethylcyclohe x-3-enecarbonitrile | spicy, clean linen |
| **54** | 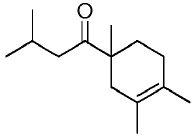 | 3-methyl-1-(1-(*R*,*S*),3,4-trimethylcyclohe x-3-enyl)butan-1-one | green, unripe fruit, malt (beer) |
| **55** | 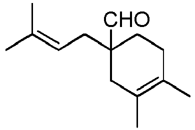 | 3,4-dimethyl-1-(*R*,*S*)-(3-methylbut-2-enyl)cyclohex-3-enecarbaldehyde | floral (geranium) |
| **56** | 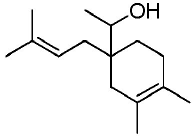 | 1-(*R*,*S*)-(3,4-dimethyl-1-(3-methylbut-2-enyl)cyclohex-3-enyl)ethanol | herbal, citrus |
| **57** | 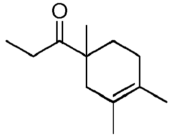 | 1-(1-(*R*,*S*),3,4-trimethylcyclohe x-3-enyl)propan-1-one | citrus, floral (fresia), herbal |
| **58** | 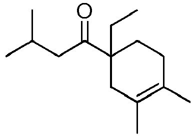 | 1-(1-(*R*,*S*)-ethyl-3,4-dimethyl-cyclohex-3-enyl)-3-methylbutan-1-one | fruity, floral, herbal |
| **59** | 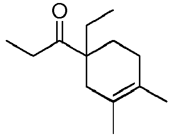 | 1-(1-(*R*,*S*)-ethyl-3,4-dimethyl-cyclohex-3-enyl)propan-1-one | herbal (coriander) |
| **60** | 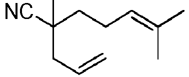 | 2-(*R*,*S*)-allyl-2,6-dimethylhept-5-enenitrile | celery, mussels, sea |
| **61** | 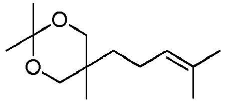 | 2,2,5-trimethyl-5-(*R*,*S*)-(4-methylpent-3-en-1-yl)-1,3-dioxane | nutty, creamy |
| **62** | 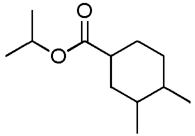 | *(R*,*S)*-isopropyl 3,4-dimethylcyclohe xanecarboxylate | fruity (banana) |
| **63** | 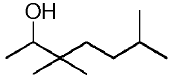 | 3,3,6-trimethylheptan-2-(*R*,*S*)-ol | herbal (lilac), citrus |
| **64** | 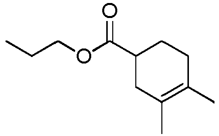 | (*R*,*S*)-propyl 3,4-dimethyl cyclohex-3-enecarboxylate | white floral |
| **65** | 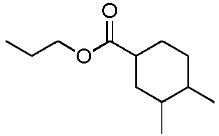 | (*R*,*S*)-propyl 3,4-dimethylcyclohe xanecarboxylate | fruity, floral |
| **66** | 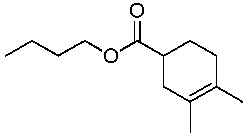 | (*R*,*S*)-butyl 3,4-dimethylcyclohe x-3-enecarboxylate | fruity, floral |
| **67** | 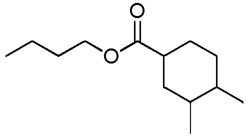 | (*R*,*S*)-butyl 3,4-dimethylcyclohe xanecarboxylate | fruity, floral |
| **68** | 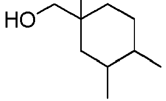 | *(R,S)*-(1,3,4-trimethylcyclohe xyl)methanol | herbal, woody, clean |
| **69** | 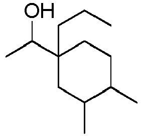 | *(R*,*S)*-1-(3,4-dimethyl-1-propylcyclohexy l)ethanol | terpenic, earthy |
| **70** | 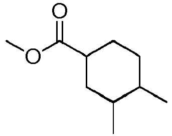 | *(R*,*S)*-methyl 3,4-dimethylcyclohe xanecarboxylate | fruity (pineapple) |
| **71** | 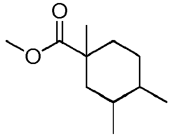 | *(R*,*S)*-methyl 1,3,4-trimethylcyclohe xanecarboxylate | fruity (grapefruit) |
| **72** | 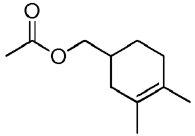 | *(R,S)-*(3,4-dimethylcyclohe x-3-enyl)methyl acetate | fruity (pear, strawberry), chewing gum |
| **73** | 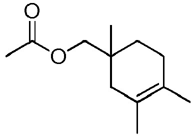 | (1-(*R*,*S*),3,4-trimethylcyclohe x-3-enyl)methyl acetate | spicy, acidic |
| **74** | 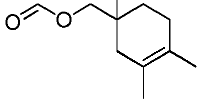 | (1-(*R*,*S*),3,4-trimethylcyclohe x-3-enyl)methyl formate | herbal |
| **75** | 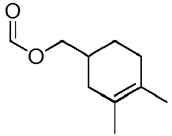 | *(R,S)*-(3,4-dimethylcyclohe x-3-enyl)methyl formate | aldehydic, fresh (mimosa) |
| **76** | 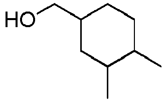 | *(R,S)*-(3,4-dimethylcyclohe xyl)methanol | green (apple) |
| **77** | 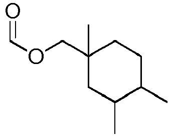 | *(R,S)*-(1,3,4-trimethyl cyclohexyl)meth yl formate | violet, floral |
| **78** | 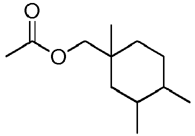 | *(R,S)*-(1,3,4-trimethyl cyclohexyl)meth yl acetate | fruity, herbal |
| **79** | 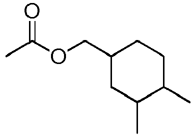 | *(R,S)*-(3,4-dimethyl cyclohexyl)meth yl acetate | rose, fruity |
| **80** | 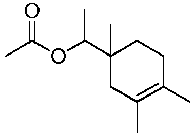 | (*R*,*S*)-1-(1,3,4-trimethylcyclohe x-3-enyl)ethyl acetate | fruity, herbal, clean |
| **81** | 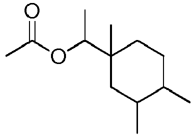 | (*R*,*S*)-1-(1,3,4-trimethylcyclohe xyl)ethyl acetate | balsamic, sweet |
| **82** | 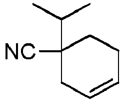 | *1-(R,S)-*isopropylcyclohe x-3-enecarbonitrile | spicy |
| **83** | 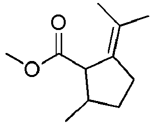 | methyl 2-(*R*,*S*)-methyl -5(propan-2-ylidene)-cyclopentanecar boxylate | fresh, peppermint |
| **84** | 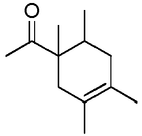 | 1-*(R,S)*-*(*1,3,4,6-*(R,S)-tetra-*methylcyclohex-3-enyl)ethanone | cedarwood |
| **85** | 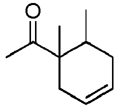 | 1-(1,6-*(R,S)-*dimethylcyclohe x-3-enyl)ethanone | woody, camphoraceo us |
| **86** | 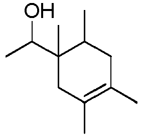 | *(R,S)*-1-(1,3,4,6-tetra-methylcyclohex-3-enyl)ethanol | lilac |
| **87** | 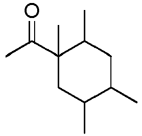 | *(R,S)*-1-(1,2,4,5-tetramethylcyclo hexyl)ethanone | woody, fruity |
| **88** | 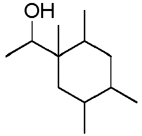 | *(R,S)*-1-(1,2,4,5-tetramethylcyclo hexyl)ethanol | sweet, fruity |
| **89** | 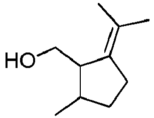 | (2-(*R*,*S*)-methyl-5-(propan-2-ylidene)cyclopen tyl)methanol | quinoline |
| **90** | 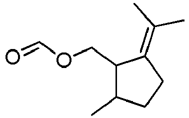 | (*R*,*S*)-(2-methyl-5-(propan-2-ylidene)cyclopen tyl)methyl formate | woody |
| **91** | 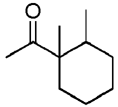 | 1-(1,2-dimethyl cyclohexyl)ethan one | woody, earthy camphoraceo us |
| **92** | 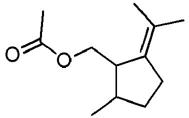 | (*R*,*S*)-(2-methyl-5-(propan-2-ylidene)cyclopen tyl)methyl acetate | woody, violet |
| **93** | 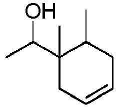 | (*R*,*S*)-1-(1,6-dimethylcyclohe x-3-enyl)ethanol | earthy (moss) |
| **94** | 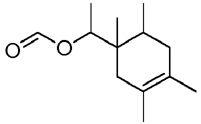 | (*R*,*S*)-1-(1,3,4,6-tetramethyl cyclohex-3-enyl)ethyl formate | violet, woody |
| **95** | 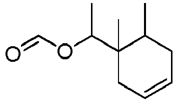 | (*R*,*S*)-1-(1,6-dimethylcyclohe x-3-enyl)ethyl formate | (soft) woody, spicy |
| **96** | 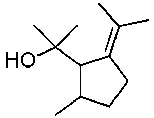 | (*R*,*S*)-2-(2-methyl-5-(propan-2-ylidene)cyclopen tyl)propan-2-ol | woody, minty |
| **97** | 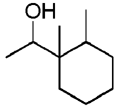 | (*R*,*S*)-1-(1,2-dimethyl cyclohexyl)ethan ol | woody, earthy |
| **98** | 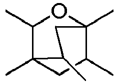 | 1,3,4,6,8-pentamethyl-2-oxa-bicyclo[2.2.2]oct ane | herbal, camphor |
| **99** | 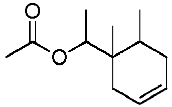 | (*R*,*S*)-1-(1,6-dimethylcyclohe x-3-enyl)ethyl acetate | violet, fruity |
| **100** | 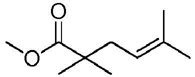 | methyl 2,2,5-trimethylhex-4-enoate | floral (chamomile), aromatic, citrus |
| **101** | 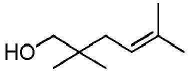 | 2,2,5-trimethylhex-4-en-1-ol | lilac, clean |
| **102** | 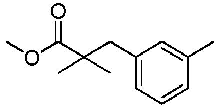 | methyl 2,2-dimethyl-3-m-tolylpropanoate | cyclamen, herbal |
| **103** | 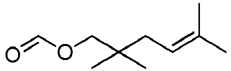 | 2,2,5-trimethylhex-4-enyl formate | citrus, gun powder |
| **104** | 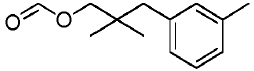 | 2,2-dimethyl-3-m-tolylpropyl formate | citrus, lemon |
| **105** | 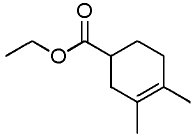 | *(R*,*S)*-ethyl 3,4-dimethylcyclohe x-3-enecarboxylate | fruity, herbal, spicy |
| **106** | 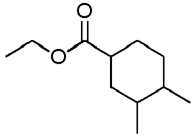 | (R,S)-ethyl 3,4-dimethylcyclohe xanecarboxylate | fruity (chewing gum), pineapple - strawberry - red apple |
| **107** | 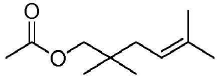 | 2,2,5-trimethylhex-4-enyl acetate | citrus, bergamot |
| **108** | 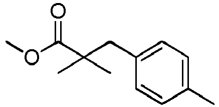 | methyl 2,2-dimethyl-3-p-tolylpropanoate | mushroom, chervil |
| **109** | 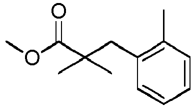 | methyl 2,2-dimethyl-3-o-tolylpropanoate | saffron, woody |
| **110** | 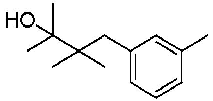 | 2,3,3-trimethyl-4-m-tolylbutan-2-ol | dark chocolate |
| **111** | 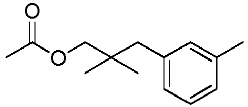 | 2,2-dimethyl-3-m-tolylpropyl acetate | citrus |
| **112** | 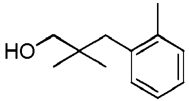 | 2,2-dimethyl-3-o-tolylpropan-1-ol | disinfectant (toilet bowl cleaner) |
| **113** | 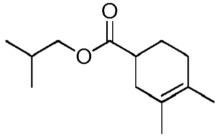 | isobutyl 3,4-dimethylcyclohe x-3-enecarboxylate | green, garlic, fruity, citrus |
| **114** | 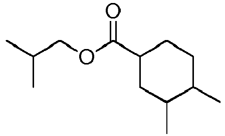 | isobutyl 3,4-dimethylcyclohe xanecarboxylate | fruity, butyric |
| **115** | 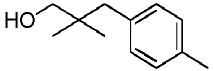 | 2,2-dimethyl-3-p-tolylpropan-1-ol | violet, minty |
| **116** | 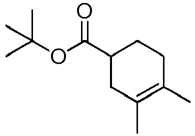 | tert-butyl 3,4-dimethylcyclohe x-3-enecarboxylate | herbal, aromatic |
| **117** | 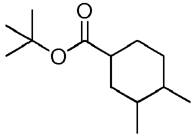 | *(R*,*S)*-tert-butyl 3,4-dimethylcyclohe xanecarboxylate | clean, ozonic |
| **118** | 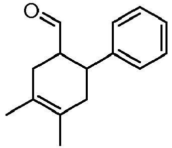 | 4,5-dimethyl-1,2,3,6-tetrahydro-[1,1'-biphenyl]-2-carbaldehyde | floral, fruity, cherry |
| **119** | 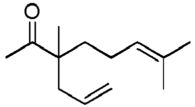 | 3-(*R*,*S*)-allyl-3,7-dimethyloct-6-en-2-one | floral |
| **120** | 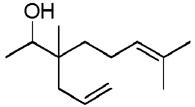 | 3-(*R*,*S*)-allyl-3,7-dimethyloct-6-en-2-(*R*,*S*)-ol | floral |
| **121** | 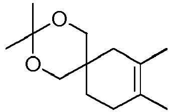 | 3,3,8,9-tetramethyl-2,4-dioxaspiro[5.5]u ndec-8-ene | green |
| **122** | 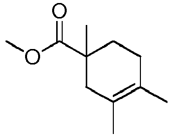 | methyl 1-(*R,S*),3,4-trimethyl-cyclohex-3-enecarboxylate | fresh, citrus, herbal (bergamot) |
| **123** | 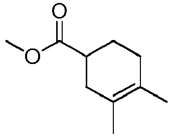 | methyl 3,4-dimethylcyclohe x-3-ene-(*R*,*S*)-carboxylate | banana skin |
| **124** | 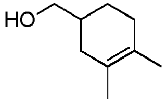 | (3,4-dimethyl-(*R*,*S*)-cyclohex-3-enyl)methanol | floral (green lily) |
| **125** | 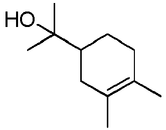 | 2-(3,4-dimethylcyclohe x-3-enyl)-(*R*,*S*)-propan-2-ol | spicy (fenugreek) |
| **126** | 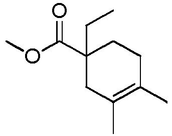 | methyl 1-(*R*,*S*)-ethyl-3,4-dimethyl-cyclohex-3-enecarboxylate | fresh, fruity, floral |
| **127** | 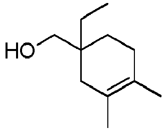 | (1-(*R*,*S*)-ethyl-3,4-dimethyl-cyclohex-3-enyl)methanol | floral, lilac |
| **128** | 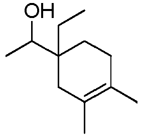 | 1-(1(*R*,*S*)-ethyl-3,4-dimethylcyclohe x-3-enyl)ethanol | floral, lilac |
| **129** | 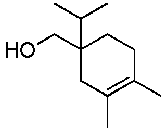 | (1-(*R*,*S*)-isopropyl-3,4-dimethyl-cyclohex-3-enyl)methanol | floral, aromatic |
| **130** | 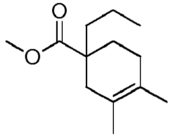 | methyl 3,4-dimethyl-1-(*R*,*S*)-propylcyclohex-3-enecarboxylate | violet, fruity, herbal |
| **131** | 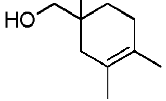 | (1-(*R*,*S*)-,3,4-trimethylcyclohe x-3-enyl)methanol | woody |
| **132** | 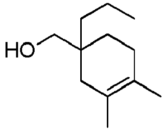 | (3,4-dimethyl-1-(*R*,*S*)-propylcyclohex-3-enyl)methanol | terpenic, pine, woody |
| **133** | 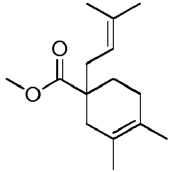 | methyl 3,4-dimethyl-1-(*R,S*)-(3-methylbut-2-enyl)cyclohex-3-enecarboxylate | citronella, clean |
| **134** | 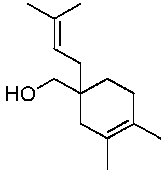 | (3,4-dimethyl-1-(*R*,*S*)-(3-methylbut-2-enyl)cyclohex-3-enyl)methanol | herbal, woody, smoky |
| **135** | 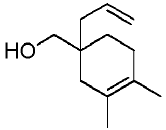 | (1-(*R*,*S*)-allyl-3,4-dimethyl-cyclohex-3-enyl)methanol | pine, indolic |
| **136** | 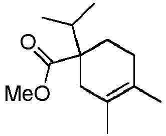 | methyl 1-(*R*,*S*)-isopropyl-3,4-dimethylcyclohe x-3-enecarboxylate | violet |
| **137** | 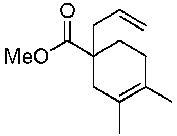 | methyl 1-(*R*,*S*)-allyl-3,4-dimethyl-cyclohex-3-enecarboxylate | green cucumber |
| **138** | 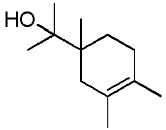 | 2-(1-(R,S)-,3,4-trimethylcyclohe x-3-enyl)propan-2-ol | floral |
| **139** | 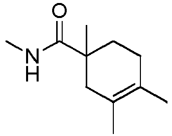 | N,1-(*R*,*S*),3,4-tetramethyl-cyclohex-3-enecarboxamide | solventy, fruity |
| **140** | 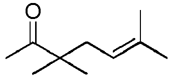 | 3,3,6-trimethylhept-5-en-2-one | citrus, fresh, grapefruit |
| **141** | 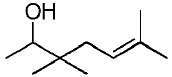 | 3,3,6-trimethylhept-5-en-2-(*R*,*S*)-ol | lime, lilac |
| **142** | 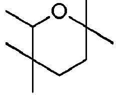 | 2,2,5,5,6-(*R*,*S*)-pentamethyl-tetrahydro-2H-pyran | camphor, green |
| **143** | 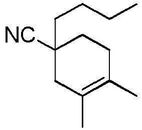 | 1-(*R,S)*-butyl-3,4-dimethyl-cyclohex-3-enecarbonitrile | floral (rose, violet), herbal |
| **144** | 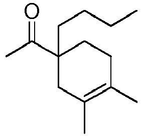 | 1-(1-*(R*,*S)*-butyl-3,4-dimethyl-cyclohex-3-enyl)ethanone | spicy |
| **145** | 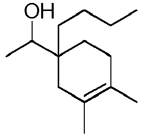 | 1-(1-*(R*,*S)*-butyl-3,4-dimethylcyclohe x-3-enyl)ethanol | violet, calamus |
| **146** | 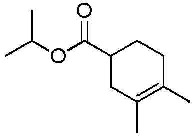 | *(R*,*S)*-isopropyl 3,4-dimethyl-cyclohex-3-enecarboxylate | white floral, fresh, aldehydic |

The PRM Molecule Number 20, 6-(R,S)-ethyl-6,9-dimethyldec-8-en-5-ol, disclosed in Table 1 above (*a.k.a.*, molecules - as referred to in the Examples section) may provide one or more of the following benefits at levels that Applicants believe are unexpected in view of PRMs in general: a cooling sensation, neat product odor; wet fabric odor when applied to a fabric; dry fabric odor when applied to a fabric; reduced leakage from an encapsulate, including an encapsulate such as a perfume microcapsule; increased head space versus neat oil in certain perfume delivery technologies; odor when used in a matrix perfume delivery that is applied to a package; neat product odor when applied to a cleaning and/or treatment composition; fine fragrance composition odor when used in a fine fragrance; dry hair odor when a composition comprising such a PRM is applied to hair; PRM bloom from a solution comprising such a PRM; and new PRM character when applied to a situs. Confirmation of such benefits can be obtained by applying standard test methodologies detailed herein. The PRMs and stereoisomers of such PRMs disclosed in Table 1 above can be made in accordance with the teachings detailed in the present specification.

The PRMs disclosed herein may have the structure of Table 1 molecules 1, 2, 3, 4, 5, 10, 11, 12, 13, 14, 15, 16, 17, 18, 19, 20, 22, 24, 26, 27, 28, 34, 35, 36, 37, 40, 41, 42, 43, 44, 45, 46, 47, 48, 49, 50, 51, 52, 53, 54, 55, 56, 57, 58, 59, 60, 61, 62, 64, 65, 67, 69, 70, 71, 73, 74, 75, 77, 79, 80, 81, 82, 84, 86, 87, 88, 93, 94, 95, 96, 97, 98, 99, 102, 104, 105, 106, 110, 111, 113, 114, 116,117, 119, 120, 121, 126, 127, 128, 129, 130, 132, 133, 134, 135, 136, 137, 139, 142, 143, 144, 145 and 146 and stereoisomers thereof. Molecule Number 20, 6-(R,S)-ethyl-6,9-dimethyldec-8-en-5-ol, disclosed in Table 1 above and stereoisomers thereof, is according to the invention. The other molecule Numbers disclosed in Table 1 above are not.

In one aspect, a perfume or sensate composition comprising, based on total perfume weight, from about 0.01% to about 50%, from about 0.1% to about 15%, from about 0.1% to about 10% or even from about 0.5% to about 10% of one or more molecules selected from the the group consisting of: 6-(*R*,*S*)-ethyl-6,9-dimethyldec-8-en-5-ol and stereoisomers thereof; and an optional solvent is disclosed.

In another aspect, 6-(*R*,*S*)-ethyl-6,9-dimethyldec-8-en-5-ol and stereoisomers thereof are suitable for use, as defined by the present specification, in consumer products at levels, based on total consumer product weight, of from about 0.0001% to about 25%, preferably from about 0.0005% to about 10%, more preferably from about 0.001% to about 5%, more preferably from about 0.005% to about 2.5%, or most preferably from 0.01% to about 1%. Such PRMs and stereoisomers thereof may be used in various combinations in the aforementioned consumer products. Preferably, a consumer product may comprise one or more PRMs selected from Table 1 molecules 1, 2, 3, 4, 5, 6, 7, 8, 10, 11, 12, 13, 14, 15, 16, 17, 18, 19, 21, 22, 23, 24, 25, 26, 27, 28, 31, 32, 34, 35, 36, 37, 38, 40, 41, 42, 43, 44, 45, 46, 47, 48, 49, 50, 51, 52, 53, 54, 55, 56, 57, 58, 59, 60, 61, 62, 63, 64, 65, 66, 67, 69, 70, 71, 73, 74, 75, 76, 77, 79, 80, 81, 82, 84, 85, 86, 87, 88, 91, 92, 93, 94, 95, 96, 97, 98, 99, 100, 102, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145 and 146 and stereoisomers thereof.

In another aspect, 6-(*R*,*S*)-ethyl-6,9-dimethyldec-8-en-5-ol and stereoisomers thereof are suitable for use, as defined by the present specification, in cleaning and/or treatment compositions at levels, based on total cleaning and treatment products weight of from about 0.0001% to about 25%, preferably from about 0.0005% to about 10%, more preferably from about 0.001% to about 5%, more preferably from about 0.005% to about 2.5%, or most preferably from 0.01% to about 1%. Such PRMs and stereoisomers thereof may be used in various combinations in the aforementioned cleaning and/ treatment compositions. Preferably, a cleaning and/or treatment composition may comprise one or more PRMs selected from Table 1 molecules 1, 2, 3, 4, 5, 6, 7, 8, 9, 10, 11, 12, 13, 14, 15, 16, 17, 18, 19, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 84, 85, 86, 87, 88, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145 and 146; and stereoisomers thereof.

In another aspect, 6-(*R*,*S*)-ethyl-6,9-dimethyldec-8-en-5-ol and stereoisomers thereof are suitable for use, as defined by the present specification, in fabric and/or hard surface cleaning and/or treatment compositions at levels, based on total fabric and/or hard surface cleaning and/or treatment composition weight of from about 0.00001% to about 25%, preferably from 0.00005% to about 10%, more preferably from 0.0001% to about 5%, more preferably from 0.0005% to about 1.0%, or most preferably from 0.001% to about 0.5%. Such PRMs and stereoisomers thereof may be used in various combinations in the aforementioned fabric and/or hard surface cleaning and/or treatment compositions. Preferably, a fabric and/or hard surface cleaning and/or treatment composition may comprise one or more PRMs selected from Table 1 molecules 1, 2, 3, 4, 5, 6, 7, 8, 9, 10, 11, 12, 13, 14, 15, 16, 17, 18, 19, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 84, 85, 86, 87, 88, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145 and 146; and stereoisomers thereof.

In another aspect, a detergent that may comprise the same level of the 6-(*R*,*S*)-ethyl-6,9-dimethyldec-8-en-5-ol and stereoisomers thereof PRM as disclosed for the aforementioned fabric and hard surface cleaning and/or treatment compositions is disclosed. Preferably, a detergent may comprise one or more PRMs selected from Table 1 molecules 1, 2, 3, 4, 5, 6, 7, 8, 9, 10, 11, 12, 13, 14, 15, 16, 17, 18, 19, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 84, 85, 86, 87, 88, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145 and 146; and stereoisomers thereof.

In another aspect, 6-(*R*,*S*)-ethyl-6,9-dimethyldec-8-en-5-ol and stereoisomers thereof are suitable for use in highly compacted consumer products, including highly compacted fabric and hard surface cleaning and/or treatment compositions. For example, the 6-(*R*,*S*)-ethyl-6,9-dimethyldec-8-en-5-ol and stereoisomers thereof PRM disclosed in Table 1 and stereoisomers thereof may be employed in solid or fluid highly compacted detergents at levels of from about 0.00001% to about 25%, preferably from 0.00005% to about 10%, more preferably from 0.0001% to about 5%, more preferably from 0.0005% to about 1.0%, or most preferably from 0.001% to about 0.5%, based on total composition weight. Such PRMs and stereoisomers thereof may be used in various combinations in the aforementioned highly compacted detergent compositions. Such highly compact detergents typically comprise a higher than normal percentage of active ingredients. Preferably, a highly compacted detergent may comprise one or more PRMs selected from Table 1 Nos. 1, 2, 3, 4, 5, 6, 7, 8, 9, 10, 11, 12, 13, 14, 15, 16, 17, 18, 19, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 84, 85, 86, 87, 88, 91, 92, 93, 94, 95, 96, 97, 98, 99, 100, 101, 102, 103, 104, 105, 106, 107, 108, 109, 110, 111, 112, 113, 114, 115, 116, 117 , 118, 119, 120, 121, 122, 123, 124, 125, 126, 127, 128, 129, 130, 131, 132, 133, 134, 135, 136, 137, 138, 139, 140, 141, 142, 143, 144, 145 and 146; and stereoisomers thereof.

### Perfume Delivery Systems

Certain perfume delivery systems, methods of making certain perfume delivery systems and the uses of such perfume delivery systems are disclosed in USPA 2007/0275866 A1. Such perfume delivery systems include:
I. Polymer Assisted Delivery (PAD): This perfume delivery technology uses polymeric materials to deliver perfume materials. Classical coacervation, water soluble or partly soluble to insoluble charged or neutral polymers, liquid crystals, hot melts, hydrogels, perfumed plastics, microcapsules, nano- and micro-latexes, polymeric film formers, and polymeric absorbents, polymeric adsorbents, etc. are some examples. PAD includes but is not limited to:
   a.) Matrix Systems: The fragrance is dissolved or dispersed in a polymer matrix or particle. Perfumes, for example, may be 1) dispersed into the polymer prior to formulating into the product or 2) added separately from the polymer during or after formulation of the product. Diffusion of perfume from the polymer is a common trigger that allows or increases the rate of perfume release from a polymeric matrix system that is deposited or applied to the desired surface (situs), although many other triggers are know that may control perfume release. Absorption and/or adsorption into or onto polymeric particles, films, solutions, and the like are aspects of this technology. Nano- or micro-particles composed of organic materials (e.g., latexes) are examples. Suitable particles include a wide range of materials including, but not limited to polyacetal, polyacrylate, polyacrylic, polyacrylonitrile, polyamide, polyaryletherketone, polybutadiene, polybutylene, polybutylene terephthalate, polychloroprene, poly ethylene, polyethylene terephthalate, polycyclohexylene dimethylene terephthalate, polycarbonate, polychloroprene, polyhydroxyalkanoate, polyketone, polyester, polyethylene, polyetherimide, polyethersulfone, polyethylenechlorinates, polyimide, polyisoprene, polylactic acid, polymethylpentene, polyphenylene oxide, polyphenylene sulfide, polyphthalamide, polypropylene, polystyrene, polysulfone, polyvinyl acetate, polyvinyl chloride, as well as polymers or copolymers based on acrylonitrile-butadiene, cellulose acetate, ethylene-vinyl acetate, ethylene vinyl alcohol, styrene-butadiene, vinyl acetate-ethylene, and mixtures thereof.

"Standard" systems refer to those that are "pre-loaded" with the intent of keeping the pre-loaded perfume associated with the polymer until the moment or moments of perfume release. Such polymers may also suppress the neat product odor and provide a bloom and/or longevity benefit depending on the rate of perfume release. One challenge with such systems is to achieve the ideal balance between 1) in-product stability (keeping perfume inside carrier until you need it) and 2) timely release (during use or from dry situs). Achieving such stability is particularly important during in-product storage and product aging. This challenge is particularly apparent for aqueous-based, surfactant-containing products, such as heavy duty liquid laundry detergents. Many "Standard" matrix systems available effectively become "Equilibrium" systems when formulated into aqueous-based products. One may select an "Equilibrium" system or a Reservoir system, which has acceptable in-product diffusion stability and available triggers for release (e.g., friction). "Equilibrium" systems are those in which the perfume and polymer may be added separately to the product, and the equilibrium interaction between perfume and polymer leads to a benefit at one or more consumer touch points (versus a free perfume control that has no polymer-assisted delivery technology). The polymer may also be pre-loaded with perfume; however, part or all of the perfume may diffuse during in-product storage reaching an equilibrium that includes having desired perfume raw materials (PRMs) associated with the polymer. The polymer then carries the perfume to the surface, and release is typically via perfume diffusion. The use of such equilibrium system polymers has the potential to decrease the neat product odor intensity of the neat product (usually more so in the case of pre-loaded standard system). Deposition of such polymers may serve to "flatten" the release profile and provide increased longevity. As indicated above, such longevity would be achieved by suppressing the initial intensity and may enable the formulator to use more high impact or low odor detection threshold (ODT) or low Kovats Index (KI) PRMs to achieve FMOT benefits without initial intensity that is too strong or distorted. It is important that perfume release occurs within the time frame of the application to impact the desired consumer touch point or touch points. Suitable micro-particles and micro-latexes as well as methods of making same may be found in USPA 2005/0003980 A1. Matrix systems also include hot melt adhesives and perfume plastics. In addition, hydrophobically modified polysaccharides may be formulated into the perfumed product to increase perfume deposition and/or modify perfume release. All such matrix systems, including for example polysaccharides and nanolatexes may be combined with other PDTs, including other PAD systems such as PAD reservoir systems in the form of a perfume microcapsule (PMC). Polymer Assisted Delivery (PAD) matrix systems may include those described in US Patent Applications 2004/0110648 A1.

Silicones are also examples of polymers that may be used as PDT, and can provide perfume benefits in a manner similar to the polymer-assisted delivery "matrix system". Such a PDT is referred to as silicone-assisted delivery (SAD). One may pre-load silicones with perfume, or use them as an equilibrium system as described for PAD. Functionalized silicones may also be used. Examples of silicones include polydimethylsiloxane and polyalkyldimethylsiloxanes. Other examples include those with amine functionality, which may be used to provide benefits associated with amine-assisted delivery (AAD) and/or polymer-assisted delivery (PAD) and/or amine-reaction products (ARP).
b.) Reservoir Systems: Reservoir systems are also known as a core-shell type technology, or one in which the fragrance is surrounded by a perfume release controlling membrane, which may serve as a protective shell. The material inside the microcapsule is referred to as the core, internal phase, or fill, whereas the wall is sometimes called a shell, coating, or membrane. Microparticles or pressure sensitive capsules or microcapsules are examples of this technology. Microcapsules of the current invention are formed by a variety of procedures that include, but are not limited to, coating, extrusion, spray-drying, interfacial, in-situ and matrix polymerization. The possible shell materials vary widely in their stability toward water. Among the most stable are polyoxymethyleneurea (PMU)-based materials, which may hold certain PRMs for even long periods of time in aqueous solution (or product). Such systems include but are not limited to urea-formaldehyde and/or melamine-formaldehyde. Stable shell materials include polyacrylate-based materials obtained as reaction product of an oil soluble or dispersible amine with a multifunctional acrylate or methacrylate monomer or oligomer, an oil soluble acid and an initiator, in presence of an anionic emulsifier comprising a water soluble or water dispersible acrylic acid alkyl acid copolymer, an alkali or alkali salt. Gelatin-based microcapsules may be prepared so that they dissolve quickly or slowly in water, depending for example on the degree of cross-linking. Many other capsule wall materials are available and vary in the degree of perfume diffusion stability observed. Without wishing to be bound by theory, the rate of release of perfume from a capsule, for example, once deposited on a surface is typically in reverse order of in-product perfume diffusion stability. As such, urea-formaldehyde and melamine-formaldehyde microcapsules for example, typically require a release mechanism other than, or in addition to, diffusion for release, such as mechanical force (e.g., friction, pressure, shear stress) that serves to break the capsule and increase the rate of perfume (fragrance) release. Other triggers include melting, dissolution, hydrolysis or other chemical reaction, electromagnetic radiation, and the like. The use of pre-loaded microcapsules requires the proper ratio of in-product stability and in-use and/or on-surface (on-situs) release, as well as proper selection of PRMs. Microcapsules that are based on urea-formaldehyde and/or melamine-formaldehyde are relatively stable, especially in near neutral aqueous-based solutions. These materials may require a friction trigger which may not be applicable to all product applications. Other microcapsule materials (e.g., gelatin) may be unstable in aqueous-based products and may even provide reduced benefit (versus free perfume control) when in-product aged. Scratch and sniff technologies are yet another example of PAD.

II. Molecule-Assisted Delivery (MAD): Non-polymer materials or molecules may also serve to improve the delivery of perfume. Without wishing to be bound by theory, perfume may non-covalently interact with organic materials, resulting in altered deposition and/or release. Non-limiting examples of such organic materials include but are not limited to hydrophobic materials such as organic oils, waxes, mineral oils, petrolatum, fatty acids or esters, sugars, surfactants, liposomes and even other perfume raw material (perfume oils), as well as natural oils, including body and/or other soils. Perfume fixatives are yet another example. In one aspect, non-polymeric materials or molecules have a CLogP greater than about 2. Molecule-Assisted Delivery (MAD) may also include those described in USP 7,119,060.

III. Fiber-Assisted Delivery (FAD): The choice or use of a situs itself may serve to improve the delivery of perfume. In fact, the situs itself may be a perfume delivery technology. For example, different fabric types such as cotton or polyester will have different properties with respect to ability to attract and/or retain and/or release perfume. The amount of perfume deposited on or in fibers may be altered by the choice of fiber, and also by the history or treatment of the fiber, as well as by any fiber coatings or treatments. Fibers may be woven and non-woven as well as natural or synthetic. Natural fibers include those produced by plants, animals, and geological processes, and include but are not limited to cellulose materials such as cotton, linen, hemp jute, flax, ramie, and sisal, and fibers used to manufacture paper and cloth. Fiber-Assisted Delivery may consist of the use of wood fiber, such as thermomechanical pulp and bleached or unbleached kraft or sulfite pulps. Animal fibers consist largely of particular proteins, such as silk, sinew, catgut and hair (including wool). Polymer fibers based on synthetic chemicals include but are not limited to polyamide nylon, PET or PBT polyester, phenol-formaldehyde (PF), polyvinyl alcohol fiber (PVOH), polyvinyl chloride fiber (PVC), polyolefins (PP and PE), and acrylic polymers. All such fibers may be pre-loaded with a perfume, and then added to a product that may or may not contain free perfume and/or one or more perfume delivery technologies. In one aspect, the fibers may be added to a product prior to being loaded with a perfume, and then loaded with a perfume by adding a perfume that may diffuse into the fiber, to the product. Without wishing to be bound by theory, the perfume may absorb onto or be adsorbed into the fiber, for example, during product storage, and then be released at one or more moments of truth or consumer touch points.

IV. Amine Assisted Delivery (AAD): The amine-assisted delivery technology approach utilizes materials that contain an amine group to increase perfume deposition or modify perfume release during product use. There is no requirement in this approach to pre-complex or pre-react the perfume raw material(s) and amine prior to addition to the product. In one aspect, amine-containing AAD materials suitable for use herein may be non-aromatic; for example, polyalkylimine, such as polyethyleneimine (PEI), or polyvinylamine (PVAm), or aromatic, for example, anthranilates. Such materials may also be polymeric or non-polymeric. In one aspect, such materials contain at least one primary amine. This technology will allow increased longevity and controlled release also of low ODT perfume notes (e.g., aldehydes, ketones, enones) via amine functionality, and delivery of other PRMs, without being bound by theory, via polymer-assisted delivery for polymeric amines. Without technology, volatile top notes can be lost too quickly, leaving a higher ratio of middle and base notes to top notes. The use of a polymeric amine allows higher levels of top notes and other PRMS to be used to obtain freshness longevity without causing neat product odor to be more intense than desired, or allows top notes and other PRMs to be used more efficiently. In one aspect, AAD systems are effective at delivering PRMs at pH greater than about neutral. Without wishing to be bound by theory, conditions in which more of the amines of the AAD system are deprotonated may result in an increased affinity of the deprotonated amines for PRMs such as aldehydes and ketones, including unsaturated ketones and enones such as damascone. In another aspect, polymeric amines are effective at delivering PRMs at pH less than about neutral. Without wishing to be bound by theory, conditions in which more of the amines of the AAD system are protonated may result in a decreased affinity of the protonated amines for PRMs such as aldehydes and ketones, and a strong affinity of the polymer framework for a broad range of PRMs. In such an aspect, polymer-assisted delivery may be delivering more of the perfume benefit; such systems are a subspecies of AAD and may be referred to as Amine- Polymer-Assisted Delivery or APAD. In some cases when the APAD is employed in a composition that has a pH of less than seven, such APAD systems may also be considered Polymer-Assisted Delivery (PAD). In yet another aspect, AAD and PAD systems may interact with other materials, such as anionic surfactants or polymers to form coacervate and/or coacervates-like systems. In another aspect, a material that contains a heteroatom other than nitrogen, for example sulfur, phosphorus or selenium, may be used as an alternative to amine compounds. In yet another aspect, the aforementioned alternative compounds can be used in combination with amine compounds. In yet another aspect, a single molecule may comprise an amine moiety and one or more of the alternative heteroatom moieties, for example, thiols, phosphines and selenols. Suitable AAD systems as well as methods of making same may be found in US Patent Applications 2005/0003980 A1.

V. Cyclodextrin Delivery System (CD): This technology approach uses a cyclic oligosaccharide or cyclodextrin to improve the delivery of perfume. Typically a perfume and cyclodextrin (CD) complex is formed. Such complexes may be preformed, formed in-situ, or formed on or in the situs. Without wishing to be bound by theory, loss of water may serve to shift the equilibrium toward the CD-Perfume complex, especially if other adjunct ingredients (e.g., surfactant) are not present at high concentration to compete with the perfume for the cyclodextrin cavity. A bloom benefit may be achieved if water exposure or an increase in moisture content occurs at a later time point. In addition, cyclodextrin allows the perfume formulator increased flexibility in selection of PRMs. Cyclodextrin may be pre-loaded with perfume or added separately from perfume to obtain the desired perfume stability, deposition or release benefit. Suitable CDs as well as methods of making same may be found in USPA 2005/0003980 A1.

VI. Starch Encapsulated Accord (SEA): The use of a starch encapsulated accord (SEA) technology allows one to modify the properties of the perfume, for example, by converting a liquid perfume into a solid by adding ingredients such as starch. The benefit includes increased perfume retention during product storage, especially under non-aqueous conditions. Upon exposure to moisture, a perfume bloom may be triggered. Benefits at other moments of truth may also be achieved because the starch allows the product formulator to select PRMs or PRM concentrations that normally cannot be used without the presence of SEA. Another technology example includes the use of other organic and inorganic materials, such as silica to convert perfume from liquid to solid. Suitable SEAs as well as methods of making same may be found in USP 6,458,754 B1.

VII. Inorganic Carrier Delivery System (ZIC): This technology relates to the use of porous zeolites or other inorganic materials to deliver perfumes. Perfume-loaded zeolite may be used with or without adjunct ingredients used for example to coat the perfume-loaded zeolite (PLZ) to change its perfume release properties during product storage or during use or from the dry situs. Suitable zeolite and inorganic carriers as well as methods of making same may be found in USPA 2005/0003980 A1. Silica is another form of ZIC. Another example of a suitable inorganic carrier includes inorganic tubules, where the perfume or other active material is contained within the lumen of the nano- or micro-tubules. In one aspect, the perfume-loaded inorganic tubule (or Perfume-Loaded Tubule or PLT) is a mineral nano- or micro-tubule, such as halloysite or mixtures of halloysite with other inorganic materials, including other clays. The PLT technology may also comprise additional ingredients on the inside and/or outside of the tubule for the purpose of improving in-product diffusion stability, deposition on the desired situs or for controlling the release rate of the loaded perfume. Monomeric and/or polymeric materials, including starch encapsulation, may be used to coat, plug, cap, or otherwise encapsulate the PLT. Suitable PLT systems as well as methods of making same may be found in USP 5,651,976.

VIII. Pro-Perfume (PP): This technology refers to perfume technologies that result from the reaction of perfume materials with other substrates or chemicals to form materials that have a covalent bond between one or more PRMs and one or more carriers. The PRM is converted into a new material called a pro-PRM (i.e., pro-perfume), which then may release the original PRM upon exposure to a trigger such as water or light. Pro-perfumes may provide enhanced perfume delivery properties such as increased perfume deposition, longevity, stability, retention, and the like. Pro-perfumes include those that are monomeric (non-polymeric) or polymeric, and may be pre-formed or may be formed in-situ under equilibrium conditions, such as those that may be present during in-product storage or on the wet or dry situs. Nonlimiting examples of pro-perfumes include Michael adducts (e.g., beta-amino ketones), aromatic or non-aromatic imines (Schiff bases), oxazolidines, beta-keto esters, and orthoesters. Another aspect includes compounds comprising one or more beta-oxy or beta-thio carbonyl moieties capable of releasing a PRM, for example, an alpha, beta-unsaturated ketone, aldehyde or carboxylic ester. The typical trigger for perfume release is exposure to water; although other triggers may include enzymes, heat, light, pH change, autoxidation, a shift of equilibrium, change in concentration or ionic strength and others. For aqueous-based products, light-triggered pro-perfumes are particularly suited. Such photo-pro-perfumes (PPPs) include but are not limited to those that release coumarin derivatives and perfumes and/or pro-perfumes upon being triggered. The released pro-perfume may release one or more PRMs by means of any of the above mentioned triggers. In one aspect, the photo-pro-perfume releases a nitrogen-based pro-perfume when exposed to a light and/or moisture trigger. In another aspect, the nitrogen-based pro-perfume, released from the photo-pro-perfume, releases one or more PRMs selected, for example, from aldehydes, ketones (including enones) and alcohols. In still another aspect, the PPP releases a dihydroxy coumarin derivative. The light-triggered pro-perfume may also be an ester that releases a coumarin derivative and a perfume alcohol. In one aspect the pro-perfume is a dimethoxybenzoin derivative as described in USPA 2006/0020459 A1. In another aspect the pro-perfume is a 3', 5'-dimethoxybenzoin (DMB) derivative that releases an alcohol upon exposure to electromagnetic radiation. In yet another aspect, the pro-perfume releases one or more low ODT PRMs, including tertiary alcohols such as linalool, tetrahydrolinalool, or dihydromyrcenol. Suitable pro-perfumes and methods of making same can be found in US Patents 7,018,978 B2In one aspect, the PRMs disclosed in Table 1 and stereoisomers thereof are suitable for use in perfume delivery systems at levels, based on total perfume delivery system weight, of from 0.001% to about 50%, preferably from 0.005% to 30%, more preferably from 0.01% to about 10%, more preferably from 0.025% to about 5%, or most preferably from 0.025% to about 1%.

In another aspect, the perfume delivery systems disclosed herein are suitable for use in consumer products, cleaning and treatment compositions, fabric and hard surface cleaning and/or treatment compositions, detergents, and highly compacted consumer products, including highly compacted fabric and hard surface cleaning and/or treatment compositions (e.g., solid or fluid highly compacted detergents) at levels, based on total consumer product weight, from about 0.001% to about 20%, preferably from about 0.01% to about 10%, more preferably from about 0.05% to about 5%, most preferably from about 0.1% to about 0.5%.

In another aspect, the amount of PRMs from Table 1 present in the perfume delivery systems, based on the total microcapsule and/or nanocapsule (Polymer Assisted Delivery (PAD) Reservoir System) weight, may be from about 0.1% to about 99%, preferably from 25% to about 95%, more preferably from 30 to about 90%, more preferably from 45% to about 90%, or most preferably from 65% to about 90%, comprising 6-(*R*,*S*)-ethyl-6,9-dimethyldec-8-en-5-ol and stereoisomers thereof. Preferably, microcapsules and/or nanocapsules may comprise one or more PRMs selected from Table 1 molecules 1, 2, 3, 4, 5, 6, 7, 8, 9, 10, 11, 12, 13, 14, 15, 16, 17, 18, 19, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117 , 118, 119, 120, 121, 122, 123, 124, 125, 126, 127, 128, 129, 130, 131, 132, 133, 134, 135, 136, 137, 138, 139, 140, 141, 142, 143, 144, 145 and 146 ; stereoisomers of Table 1 molecules 1, 2, 3, 4, 5, 6, 7, 8, 9, 10, 11, 12, 13, 14, 15, 16, 17, 18, 19, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117 , 118, 119, 120, 121, 122, 123, 124, 125, 126, 127, 128, 129, 130, 131, 132, 133, 134, 135, 136, 137, 138, 139, 140, 141, 142, 143, 144, 145 and 146 and mixtures thereof.

In one aspect, the amount of total perfume based on total weight of starch encapsulates and starch agglomerates (Starch Encapsulated Accord (SEA)) ranges from 0.1% to about 99%, preferably from 25% to about 95%, more preferably from 30 to about 90%, more preferably from 45% to about 90%, or most preferably from 65% to about 90% comprising 6-(*R*,*S*)-ethyl-6,9-dimethyldec-8-en-5-ol and stereoisomers thereof. In one aspect, the PRMs disclosed in Table 1 and stereoisomers thereof comprising 6-(*R*,*S*)-ethyl-6,9-dimethyldec-8-en-5-ol and stereoisomers thereof are suitable for use in such starch encapsulates and starch agglomerates. Such PRMs and stereoisomers thereof may be used in combination in such starch encapsulates and starch agglomerates.

In another aspect, the amount of total perfume based on total weight of [cyclodextrin - perfume] complexes (Cyclodextrin (CD)) ranges from 0.1% to about 99%, preferably from 2.5% to about 75%, more preferably from 5% to about 60%, more preferably from 5% to about 50%, most preferably from 5% to about 25% comprising 6-(*R*,*S*)-ethyl-6,9-dimethyldec-8-en-5-ol and stereoisomers thereof. In one aspect, the PRMs disclosed in Table 1 comprising 6-(*R*,*S*)-ethyl-6,9-dimethyldec-8-en-5-ol and stereoisomers thereof are suitable for use in such [cyclodextrin - perfume] complexes. Such PRMs and stereoisomers thereof may be used in combination in such [cyclodextrin - perfume] complexes.

In another aspect, the amount of total perfume based on total weight of Polymer Assisted Delivery (PAD) Matrix Systems (including Silicones) ranges from 0.1% to about 99%, preferably from 2.5% to about 75%, more preferably from 5% to about 60%, more preferably from 5% to about 50%, most preferably from 5% to about 25% comprising 6-(*R*,*S*)-ethyl-6,9-dimethyldec-8-en-5-ol and stereoisomers thereof. In one aspect, the amount of total perfume based on total weight of a hot melt perfume delivery system/perfume loaded plastic Matrix System and ranges from 1% to about 99%, preferably from 2.5% to about 75%, more preferably from 5% to about 60%, more preferably from 5% to about 50%, most preferably from 10 % to about 50% comprising 6-(*R*,*S*)-ethyl-6,9-dimethyldec-8-en-5-ol and stereoisomers thereof. In one aspect, the PRMs disclosed in Table 1 and stereoisomers thereof comprising 6-(*R*,*S*)-ethyl-6,9-dimethyldec-8-en-5-ol and stereoisomers thereof are suitable for use in such Polymer Assisted Delivery (PAD) Matrix Systems, including hot melt perfume delivery system/perfume loaded plastic Matrix Systems. Such PRMs and stereoisomers thereof may be used in various combinations in such Polymer Assisted Delivery (PAD) Matrix Systems (including hot melt perfume delivery system/perfume loaded plastic Matrix Systems).

In one aspect, the amount of total perfume based on total weight of Amine Assisted Delivery (AAD) (including Aminosilicones) ranges from 1% to about 99%, preferably from 2.5% to about 75%, more preferably from 5% to about 60%, more preferably from 5% to about 50%, most preferably from 5% to about 25% comprising 6-(*R*,*S*)-ethyl-6,9-dimethyldec-8-en-5-ol and stereoisomers thereof. In one aspect, the PRMs disclosed in Table 1 and stereoisomers thereof comprising 6-(*R*,*S*)-ethyl-6,9-dimethyldec-8-en-5-ol and stereoisomers thereof are suitable for use in such Amine Assisted Delivery (AAD) systems. Such PRMs and stereoisomers thereof may be used in various combinations in such Amine Assisted Delivery (AAD) systems. Preferably, an Amine Assisted Delivery (AAD) system may comprise one or more PRMs selected from Table 1 molecules 1, 3, 10, 12, 13, 17, 19, 23, 26, 27, 29, 30, 31, 32, 33, 34, 35, 36, 37, 38, 39, 41, 42, 46, 48, 50, 51, 52, 54, 55, 57, 58, 59, 84, 85, 87, 91, 118, 119, 140 and 144; stereoisomers of Table 1 molecules 1, 3, 10, 12, 13, 17, 19, 23, 26, 27, 29, 30, 31, 32, 33, 34, 35, 36, 37, 38, 39, 41, 42, 46, 48, 50, 51, 52, 54, 55, 57, 58, 59, 84, 85, 87, 91, 118, 119, 140 and 144; and mixtures thereof.

PRM Nos. 1, 3, 10, 12, 13, 17, 19, 23, 26, 27, 31, 32, 34, 35, 36, 37, 41, 42, 46, 48, 52, 54, 57, 58, 59, 84, 85, 87, 91, 119, 140 and 144 are ketones. PRM Nos. 29, 30, 33, 38, 39, 50, 51, 55 and 118 are aldehydes.

In one aspect, a Pro-Perfume (PP) Amine Reaction Product (ARP) system comprising 6-(*R*,*S*)-ethyl-6,9-dimethyldec-8-en-5-ol and stereoisomers thereof may comprise one or more PRMs selected from Table 1 molecules 1, 3, 6, 10, 12, 13, 14, 15, 17, 18, 19, 23, 25, 26, 27, 29, 30, 31, 32, 33, 34, 35, 36, 37, 38, 39, 41, 42, 43, 46, 47, 48, 49, 50, 51, 52, 54, 55, 56, 57, 58, 59, 63, 68, 69, 76, 84, 85, 86, 87, 88, 89, 91, 93, 96, 97, 101, 110, 112, 115, 118, 119, 120, 124, 125, 127, 128, 129, 131, 132, 134, 135, 138, 140, 141, 144 and 145; and mixtures thereof.

PRM Nos. 1, 3, 10, 12, 13, 17, 19, 23, 26, 27, 31, 32, 34, 35, 36, 37, 41, 42, 46, 48, 52, 54, 57, 58, 59, 84, 85, 87, 91, 119, 140 and 144 are ketones. PRM Nos. 29, 30, 33, 38, 39, 50, 51, 55 and 118 are aldehydes. PRMs Nos. 6, 14, 15, 18, 20, 25, 43, 47, 49, 56, 63, 68, 69, 76, 86, 88, 89, 93, 96, 97, 101, 110, 112, 115, 120,124,125,127,128,129,131,132,134,135,138,141 and 145 are alcohols.

In one aspect, the amount of total perfume based on total weight of Pro-Perfume (PP) Amine Reaction Product (ARP) system ranges from 0.1% to about 99%, preferably from about 1% to about 99%, more preferably from 5% to about 90%, more preferably from 10% to about 75%, more preferably from 20% to about 75%, most preferably from 25% to about 60% comprising 6-(*R*,*S*)-ethyl-6,9-dimethyldec-8-en-5-ol and stereoisomers thereof.

The perfume delivery technologies (a.k.a., perfume delivery systems) that are disclosed in the present specification may be used in any combination in any type of consumer product, cleaning and/or treatment composition, fabric and hard surface cleaning and/or treatment composition, detergent, and/or highly compact detergent.

### Perfumes

The PRM Molecule Number 20, 6-(*R*,*S*)-ethyl-6,9-dimethyldec-8-en-5-ol and stereoisomers thereof, disclosed in Table 1 may be used to formulate perfumes. Such perfumes are combinations of PRMs that may comprise a combination of Table 1 PRMs comprising Molecule Number 20, 6-(*R*,*S*)-ethyl-6,9-dimethyldec-8-en-5-ol and stereoisomers thereof, or one or more Table 1 PRMs comprising Molecule Number 20, 6-(*R*,*S*)-ethyl-6,9-dimethyldec-8-en-5-ol and stereoisomers thereof, and one or more additional PRMs. When used in a perfume, the Table 1 PRMs comprising Molecule Number 20, 6-(*R*,*S*)-ethyl-6,9-dimethyldec-8-en-5-ol and stereoisomers thereof, may be employed, based on total perfume weight, at levels of from about 0.01% to about 50%, preferably from about 0.1% to about 15%, more preferably from about 0.1% to about 10% or most preferably from about 0.5% to about 10%. Such perfumes may be utilized in various applications, including being applied neat to a situs or used in a consumer product, cleaning and/or treatment composition, fabric and hard surface cleaning and/or treatment composition, detergent, and/or a highly compact detergent.

### Adjunct Materials

For the purposes of the present invention, the non-limiting list of adjuncts illustrated hereinafter are suitable for use in the compositions detailed herein (e.g., consumer products, cleaning and/or treatment compositions, fabric and hard surface cleaning and/or treatment compositions, detergents, and/or a highly compact detergents). Such adjunct materials may be desirably incorporated in certain embodiments of the compositions, for example to assist or enhance performance of the composition, for treatment of the substrate to be cleaned, or to modify the aesthetics of the composition as is the case with perfumes, colorants, dyes or the like. It is understood that such adjuncts are in addition to the components that are supplied via Applicants' perfumes and/or perfume systems detailed herein. The precise nature of these additional components, and levels of incorporation thereof, will depend on the physical form of the composition and the nature of the operation for which it is to be used.

Suitable adjunct materials include, but are not limited to, surfactants, builders, chelating agents, dye transfer inhibiting agents, dispersants, enzymes, and enzyme stabilizers, catalytic materials, bleach activators, polymeric dispersing agents, clay soil removal/anti-redeposition agents, brighteners, suds suppressors, dyes, additional perfume and perfume delivery systems, structure elasticizing agents, fabric softeners, carriers, hydrotropes, processing aids and/or pigments, metal salts, structurants or binders, anti-tartar agents, anti-caries agents, abrasives, fillers, humectants, breath agents, flavors, antibacterial agents. In addition to the disclosure below, suitable examples of such other adjuncts and levels of use are found in U.S. Patent No. 6,326,348 B1.

Each adjunct ingredient is not essential to Applicants' compositions. Thus, certain embodiments of Applicants' compositions may not contain one or more of the following adjuncts materials: bleach activators, surfactants, builders, chelating agents, dye transfer inhibiting agents, dispersants, enzymes, and enzyme stabilizers, catalytic metal complexes, polymeric dispersing agents, clay and soil removal/anti-redeposition agents, brighteners, suds suppressors, dyes, additional perfumes and perfume delivery systems, structure elasticizing agents, fabric softeners, carriers, hydrotropes, processing aids and/or pigments, metal salts, structurants or binders, anti-tartar agents, anti-caries agents, abrasives, fillers, humectants, breath agents, flavors, antibacterial agents. However, when one or more adjuncts are present, such adjuncts may be present as detailed below:
Surfactants - The compositions according to the present invention can comprise a surfactant or surfactant system wherein the surfactant can be selected from nonionic and/or anionic and/or cationic surfactants and/or ampholytic and/or zwitterionic and/or semi-polar nonionic surfactants. The surfactant is typically present at a level of from about 0.1%, from about 1%, or even from about 5% by weight of the cleaning compositions to about 99.9%, to about 80%, to about 35%, or even to about 30% by weight of the cleaning compositions.
Builders - The compositions of the present invention can comprise one or more detergent builders or builder systems. When present, the compositions will typically comprise at least about 1% builder, or from about 5% or 10% to about 80%, 50%, or even 30% by weight, of said builder. Builders include, but are not limited to, the alkali metal, ammonium and alkanolammonium salts of polyphosphates, alkali metal silicates, alkaline earth and alkali metal carbonates, aluminosilicate builders polycarboxylate compounds. ether hydroxypolycarboxylates, copolymers of maleic anhydride with ethylene or vinyl methyl ether, 1,3,5-trihydroxybenzene-2,4,6-trisulphonic acid, and carboxymethyl-oxysuccinic acid, the various alkali metal, ammonium and substituted ammonium salts of polyacetic acids such as ethylenediamine tetraacetic acid and nitrilotriacetic acid, as well as polycarboxylates such as mellitic acid, succinic acid, oxydisuccinic acid, polymaleic acid, benzene 1,3,5-tricarboxylic acid, carboxymethyloxysuccinic acid, and soluble salts thereof.
Chelating Agents - The compositions herein may also optionally contain one or more copper, iron and/or manganese chelating agents. If utilized, chelating agents will generally comprise from about 0.1% by weight of the compositions herein to about 15%, or even from about 3.0% to about 15% by weight of the compositions herein.

Dye Transfer Inhibiting Agents - The compositions of the present invention may also include one or more dye transfer inhibiting agents. Suitable polymeric dye transfer inhibiting agents include, but are not limited to, polyvinylpyrrolidone polymers, polyamine N-oxide polymers, copolymers of N-vinylpyrrolidone and N-vinylimidazole, polyvinyloxazolidones and polyvinylimidazoles or mixtures thereof. When present in the compositions herein, the dye transfer inhibiting agents are present at levels from about 0.0001%, from about 0.01%, from about 0.05% by weight of the cleaning compositions to about 10%, about 2%, or even about 1% by weight of the cleaning compositions.

Dispersants - The compositions of the present invention can also contain dispersants. Suitable water-soluble organic materials are the homo- or co-polymeric acids or their salts, in which the polycarboxylic acid may comprise at least two carboxyl radicals separated from each other by not more than two carbon atoms.

Enzymes - The compositions can comprise one or more detergent enzymes which provide cleaning performance and/or fabric care benefits. Examples of suitable enzymes include, but are not limited to, hemicellulases, peroxidases, proteases, cellulases, xylanases, lipases, phospholipases, esterases, cutinases, pectinases, keratanases, reductases, oxidases, phenoloxidases, lipoxygenases, ligninases, pullulanases, tannases, pentosanases, malanases, β-glucanases, arabinosidases, hyaluronidase, chondroitinase, laccase, and amylases, or mixtures thereof. A typical combination is a cocktail of conventional applicable enzymes like protease, lipase, cutinase and/or cellulase in conjunction with amylase.

Enzyme Stabilizers - Enzymes for use in compositions, for example, detergents can be stabilized by various techniques. The enzymes employed herein can be stabilized by the presence of water-soluble sources of calcium and/or magnesium ions in the finished compositions that provide such ions to the enzymes.

Catalytic Metal Complexes - Applicants' compositions may include catalytic metal complexes. One type of metal-containing bleach catalyst is a catalyst system comprising a transition metal cation of defined bleach catalytic activity, such as copper, iron, titanium, ruthenium, tungsten, molybdenum, or manganese cations, an auxiliary metal cation having little or no bleach catalytic activity, such as zinc or aluminum cations, and a sequestrate having defined stability constants for the catalytic and auxiliary metal cations, particularly ethylenediaminetetraacetic acid, ethylenediaminetetra (methyl-enephosphonic acid) and water-soluble salts thereof. Such catalysts are disclosed in U.S. patent 4,430,243.

If desired, the compositions herein can be catalyzed by means of a manganese compound. Such compounds and levels of use are well known in the art and include, for example, the manganese-based catalysts disclosed in U.S. patent 5,576,282. Cobalt bleach catalysts useful herein are known, and are described, for example, in U.S. patents 5,597,936.

Compositions herein may also suitably include a transition metal complex of a macropolycyclic rigid ligand - abbreviated as "MRL". As a practical matter, and not by way of limitation, the compositions and cleaning processes herein can be adjusted to provide on the order of at least one part per hundred million of the benefit agent MRL species in the aqueous washing medium, and may provide from about 0.005 ppm to about 25 ppm, from about 0.05 ppm to about 10 ppm, or even from about 0.1 ppm to about 5 ppm, of the MRL in the wash liquor. Suitable transition-metals in the instant transition-metal bleach catalyst include manganese, iron and chromium. Suitable MRL's herein are a special type of ultra-rigid ligand that is cross-bridged such as 5,12-diethyl-1,5,8,12-tetraazabicyclo[6.6.2]hexa-decane. Suitable transition metal MRLs are readily prepared by known procedures, such as taught for example in and U.S. patent 6,225,464.

### Methods of Use

Some of the consumer products disclosed herein can be used to clean or treat a situs *inter alia* a surface or fabric. Typically at least a portion of the situs is contacted with an embodiment of Applicants' composition, in neat form or diluted in a liquor, for example, a wash liquor and then the situs may be optionally washed and/or rinsed. In one aspect, a situs is optionally washed and/or rinsed, contacted with a composition according to the present invention and then optionally washed and/or rinsed. For purposes of the present invention, washing includes but is not limited to, scrubbing, and mechanical agitation. The fabric may comprise most any fabric capable of being laundered or treated in normal consumer use conditions. Liquors that may comprise the disclosed compositions may have a pH of from about 3 to about 11.5. Such compositions are typically employed at concentrations of from about 500 ppm to about 15,000 ppm in solution. When the wash solvent is water, the water temperature typically ranges from about 5 °C to about 90 °C and, when the situs comprises a fabric, the water to fabric ratio is typically from about 1:1 to about 30:1.

### EXAMPLES

### Synthesis of Table 1 Molecule Number 20

| Compound Number | Carbonyl Compound | R¹ | R² | R³ | R⁴ | R⁵ | R⁶ | R⁷ | R⁸ | R⁹ | R¹⁰ |
|---|---|---|---|---|---|---|---|---|---|---|---|
| 20 | 19 | Me | Et | 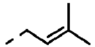 | n-Bu | n-Bu | | | | | |

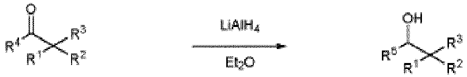

### Example 2: Preformed Amine Reaction Product

The following ingredients are weighted off in a glass vial:
1. 50% of the perfume material comprising one or more Table 1 PRMs
2. 50% of Lupasol WF (CAS# 09002-98-6) from BASF, is put at 60°C in warm water bath for 1 hour before use.

Mixing of the two ingredients is done by using the Ultra-Turrax T25 Basic equipment (from IKA) during 5 minutes. When the mixing is finished the sample is put in a warm water bath at 60°C for ± 12 hours. A homogenous, viscous material is obtained.

In the same way as described above different ratios between the components can be used:

| Weight % | | | | | |
|---|---|---|---|---|---|
| Perfume Material | 40 | 50 | 60 | 70 | 80 |
| Lupasol WF | 60 | 50 | 40 | 30 | 20 |

### Example 3: 84wt% Core / 16wt% Wall Melamine Formaldehyde (MF) Capsule (PAD Reservoir System

17 grams of butyl acrylate-acrylic acid copolymer emulsifier (Colloid C351, 25% solids, pka 4.5-4.7, (Kemira Chemicals, Inc. Kennesaw, Ga. U.S.A.) and 17 grams of polyacrylic acid (35% solids, pKa 1.5-2.5, Aldrich) are dissolved and mixed in 200 grams deionized water. The pH of the solution is adjusted to pH of 6.0with sodium hydroxide solution. 7grams of partially methylated methylol melamine resin (Cymel 385, 80% solids, (Cytec Industries West Paterson, N.J., U.S.A.)) is added to the emulsifier solution. 200 grams of perfume oil is added to the previous mixture under mechanical agitation and the temperature is raised to 45° C. After mixing at higher speed until a stable emulsion is obtained, the second solution and 4 grams of sodium sulfate salt are added to the emulsion. This second solution contains 3 grams of polyacrylic acid polymer (Colloid C121, 25% solids (Kemira Chemicals, Inc. Kennesaw, Ga. U.S.A.), 100 grams of distilled water, sodium hydroxide solution to adjust pH to 6.0, 10 grams of partially methylated methyol melamine resin (Cymel 385, 80% Cytec). This mixture is heated till 85C and maintained 8 hours with continuous stirring to complete the encapsulation process. 23 grams of acetoacetamide (Sigma-Aldrich, Saint Louis, Mo. U.S.A.) is added to the suspension. Salts and structuring agents can then still be added to the slurry.

### Example 4: Process of Making a Polymer Assisted Delivery (PAD) Matrix System

A mixture comprising 50% of a perfume composition comprising one or more Table 1 PRMs, 40% of carboxyl-terminated Hypro^{™} RLP 1300X18 (CAS#0068891-50-9) from nanoresins, (put at 60°C in warm water bath for 1 hour before mixing) and 10% of Lupasol^{®} WF(CAS# 09002-98-6) from BASF ( put at 60°C in warm water bath for 1 hour before mixing). Mixing is achieved by mixing for five minutes using a Ultra-Turrax T25 Basic equipment (from IKA). After mixing, the mixture is put in a warm water bath at 60°C for ± 12 hours. A homogenous, viscous and sticky material is obtained.

In the same way as described above different ratios between the components can be used:

| Weight % | | | | | |
|---|---|---|---|---|---|
| Perfume composition | 40 | 50 | 60 | 70 | 80 |
| Lupasol^{®} WF | 12 | 10 | 8 | 6 | 4 |
| Hypro^{™} RLP CTBN1300X18 | 48 | 40 | 32 | 24 | 16 |

| Weight % | | | | | | | | |
|---|---|---|---|---|---|---|---|---|
| Perfume composition | 50 | 50 | 50 | 50 | 50 | 50 | 50 | 50 |
| Lupasol^{®} WF | 2.5 | 5 | 7.5 | 10 | 12.5 | 15 | 17.5 | 20 |
| Hypro^{™} RLP 1300X18 | 47.5 | 45 | 42.5 | 40 | 37.5 | 35 | 32.5 | 30 |

### Example 32: Shampoo Formulations

| Ingredient | |
|---|---|
| Ammonium Laureth Sulfate (AE₃S) | 6.00 |
| Ammonium Lauryl Sulfate (ALS) | 10.00 |
| Laureth-4 Alcohol | 0.90 |
| Trihydroxystearin ⁽⁷⁾ | 0.10 |
| Perfume comprising one or more PRMs from Table 1 | 0.60 |
| Sodium Chloride | 0.40 |
| Citric Acid | 0.04 |
| Sodium Citrate | 0.40 |
| Sodium Benzoate | 0.25 |
| Ethylene Diamine Tetra Acetic Acid | 0.10 |
| Dimethicone ^{(9, 10, 11)} | 1.00 ⁽⁹⁾ |
| Water and Minors (QS to 100%) | Balance |

### Example 33 - 35 : Fine Fragrance Formulations

| Ingredient | 33 | 34 | 35 |
|---|---|---|---|
| Cyclic oligosaccharide | 0 | 5 | 10 |
| Ethanol | 90 | 75 | 80 |
| Perfume comprising one or more PRMs from Table 1 | 10 | 20 | 10 |

### Example 36- 49: Dentifrice Containing Sensate

| Ingredient | 36 | 37 | 38 | 39 | 40 | 41 | 42 | 43 | 44 |
|---|---|---|---|---|---|---|---|---|---|
| Calcium Peroxide | | | 0.1 | | | | | | |
| Carbomer 956 | 0.2 | | | 0.3 | 0.2 | 0.2 | 0.2 | 0.2 | 0.2 |
| CMC | | 0.75 | 0.2 | | | 1.0 | 1.0 | 1.0 | 1.0 |
| Color Solution (1%) | 0.05 | 0.05 | 0.5 | 0.75 | 0.18 | 0.02 | 0.25 | 0.05 | 0.05 |
| Wintergreen Spice Flavor | | | | | 0.15 | | | | |
| Fruit Mint Flavor | | 0.55 | | | | | | | |
| Mint Flavor | 0.59 | | 0.45 | | 0.42 | 1.0 | 1.2 | 1.0 | 1.0 |
| Cinnamon Flavor | | | | 0.5 | | | | | |
| Vanillyl Butyl Ether | | | | | 0.02 | | | | |
| WS-23 | | | 0.1 | 0.05 | 0.1 | | | | |
| WS-3 | | | 0.2 | 0.05 | 0.2 | | | | |
| MGA | | | | 0.2 | | | | | |
| Menthol | 0.52 | 0.55 | 0.56 | 0.15 | 0.58 | | | | |
| Sensate comprising one or more PRMs from Table 1 | 0.01 | 0.03 | 0.015 | 0.004 | 0.01 | 0.01 | 0.03 | 0.008 | 0.02 |
| Potassium Sorbate | | | | | | 0.004 | 0.008 | 0.004 | 0.004 |
| Poloxamer 407 | | | 1.0 | | 0.2 | 0.2 | 0.2 | 0.2 | 0.2 |
| Polyethylene Glycol 300 | 3.0 | 3.0 | | 3.0 | | | | | |
| Polyethylene Glycol 600 | | | 2.3 | | | | | | |
| Propylene Glycol | | | 10.0 | | | | | | |
| Saccharin Sodium | 0.46 | 0.5 | 0.45 | 0.4 | 0.58 | 0.4 | 0.4 | 0.4 | 0.4 |
| Sucralose | | | | | | | 0.02 | 0.02 | 0.02 |
| Silica Abrasive | 22.0 | 31.0 | 20.0 | 21.0 | 17.0 | 15.0 | 15.0 | 15.0 | 15.0 |
| Sodium Benzoate | | | | | | 0.004 | 0.004 | 0.004 | 0.004 |
| Silica Thickening | | | 2.0 | | | 7.0 | 7.0 | 7.0 | 7.0 |
| Sodium Bicarbonate | | 1.5 | 9.0 | | | | | | |
| Sodium Carbonate | | 0.5 | | | | | | | |
| NaOH 50% soln | | | 1.74 | 2.2 | | 2.0 | 2.0 | 2.0 | 2.0 |
| Na Lauryl Sulfate (27.9% soln) | 4.0 | 5.0 | 3.0 | 4.0 | 4.0 | | | 3.0 | 2.0 |
| Stannous Fluoride | 0.454 | 0.454 | | | | | | | |
| Sodium Fluoride | | | | | | 0.243 | 0.243 | 0.243 | |
| Sodium MFP | | | 0.76 | 0.76 | 0.76 | 0.76 | | | |
| Glycerin USP 99.7% | 9.0 | 11.9 | 33.0 | 9.0 | | | | | |
| Sorbitol Soln USP | 24.3 | 24.5 | 4.0 | 44.7 | 56.9 | 43.0 | 43.0 | 40.0 | 38.0 |
| Tetra Na Pyrophosphate Anhydrous | 2.05 | 5.045 | 3.85 | | 3.85 | | | | |
| Tetra Potassium | 6.38 | | | | | | | | |
| Pyrophosphate (60%Soln) | | | | | | | | | |
| Na Acid Pyrophosphate | 2.1 | | | 4.0 | 1.0 | 4.3 | 4.5 | 4.5 | 2.0 |
| Alkyl Phosphate | | | | | | 3.5 | 6.7 | 3.5 | 3.5 |
| Cocamidopropyl Betaine (30%Soln) | | | | | | 3.5 | | | |
| Titanium Dioxide | 0.5 | | 1.0 | | 0.25 | 0.3 | 0.3 | 0.2 | 0.2 |
| Ti02/Carnauba Wax Prills | | 0.6 | | 0.3 | | | | | |
| Xanthan Gum | 0.6 | | 0.4 | 0.45 | 0.7 | 0.3 | 0.3 | 0.3 | 0.3 |
| Water Purified USP | QS | QS | QS | QS | QS | QS | QS | QS | QS |
| | | | | | | | | | |
| Ingredient | 45 | 46 | 47 | 48 | 49 | | | | |
| Calcium Carbonate | | | | 40.0 | | | | | |
| Dibasic Calcium Phosphate Dihydrate | | | 35.0 | | | | | | |
| Silica Abrasive | 24.0 | 12.5 | | | 17.0 | | | | |
| Phytic Acid | | 0.8 | | | 2.0 | | | | |
| Gantrez S-97 | | | 2.0 | | | | | | |
| Color Solution (1%) | 0.05 | 0.05 | | | 0.05 | | | | |
| Saccharin sodium | 0.47 | 0.25 | 0.3 | 0.3 | 0.58 | | | | |
| Spice Mint Flavor | | | | 1.0 | | | | | |
| Wintergreen Spice Flavor | | 1.2 | | | 0.15 | | | | |
| Mint Flavor | | 0.3 | 0.6 | 0.5 | 0.42 | | | | |
| Cinnamon Flavor | 0.18 | | | | | | | | |
| WS-23 Coolant | 0.03 | | | | 0.02 | | | | |
| WS-3 Coolant | 0.03 | | | | 0.02 | | | | |
| MGA | 0.08 | 0.08 | | | | | | | |
| Menthol | 0.38 | 0.24 | 0.2 | 0.5 | 0.58 | | | | |
| Sensate comprising one or more PRMs from Table 1 | 0.08 | 0.005 | 0.004 | 0.008 | 0.01 | | | | |
| Glycerin | 16.5 | | 15.0 | | | | | | |
| Sorbitol solution | 10.5 | 33.0 | 11.5 | 14.0 | 57.0 | | | | |
| Poloxamer 407 | | | | | 0.2 | | | | |
| Polyethylene Glycol 300 | | | | 2.5 | | | | | |
| Polyethylene Glycol 600 | | | 3.0 | | | | | | |
| Carbomer | | 0.3 | | | 0.2 | | | | |
| CMC 7M8SF | 1.0 | 1.0 | 1.0 | 1.0 | | | | | |
| HEC 250MX | | 0.5 | | | | | | | |
| Sodium Lauryl Sulfate (27.9% soln) | 7.5 | 7.0 | 5.5 | 7.0 | 4.0 | | | | |
| NaOH 50% soln | | 1.0 | | | | | | | |
| Sodium Monofluorophosphate | 0.76 | | 0.76 | 0.76 | 0.76 | | | | |
| Sodium Fluoride | | 0.32 | | | | | | | |
| Sodium Gluconate | | 1.0 | | | | | | | |
| Stannous Chloride Dihydrate | | 1.0 | | | | | | | |
| Zinc Citrate | | 0.5 | | | | | | | |
| Potassium Nitrate | 5.0 | | | | | | | | |
| Sodium Phosphate, Tribasic | 3.2 | | | | | | | | |
| Tetra Sodium Pyrophosphate, Anhydrous | | | 0.5 | 0.5 | 3.85 | | | | |
| Sodium Acid Pyrophosphate | | | | | 1.0 | | | | |
| Titanium Dioxide | 0.5 | 0.5 | | | 0.25 | | | | |
| Xanthan Gum (Keltro 1000 | | | | 0.5 | 0.7 | | | | |
| Carrageenan | | 0.5 | | | | | | | |
| Water, Purified, USP | QS | QS | QS | QS | QS | | | | |

### Example 50-52: Mouthrinse Containing Sensate

| Ingredient | 50 | 51 | 52 |
|---|---|---|---|
| Ethanol USP 190 proof | 15 | 15 | 15 |
| Glycerin | 7.5 | 7.5 | 7.5 |
| Polysorbate 80 NF | 0.12 | 0.12 | 0.12 |
| Flavor' | 0.16 | 0.16 | 0.16 |
| Saccharin sodium | 0.067 | 0.067 | 0.06 |
| Color solution | 0.04 | 0.04 | 0.04 |
| Sensate comprising one or more PRMs from Table 1 | 0.03 | 0.017 | 1 |
| Calcium chloride | 0.025 | 0.025 | 0.025 |
| Cetylpyridinium chloride | 0.045 | 0.045 | 0.045 |
| Benzoic acid | 0.005 | 0.005 | 0.005 |
| Sodium benzoate | 0.054 | 0.054 | 0.054 |
| Water | QS | QS | QS |

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm".

## Claims

1. A molecule selected from the group consisting of: 6-(*R*,*S*)-ethyl-6,9-dimethyldec-8-en-5-ol and stereoisomers thereof.

2. A perfume or sensate composition comprising, based on total perfume weight, from 0.01% to 50%, preferably from 0.1% to 15%, more preferably from 0.1% to 10%, most preferably from 0.5% to 10% of one or more molecules selected from the group consisting of: 6-(*R*,*S*)-ethyl-6,9-dimethyldec-8-en-5-ol and stereoisomers thereof; and mixtures thereof; and an optional solvent.

3. A consumer product comprising, based on total consumer product weight, from 0.0001% to 25%, preferably from 0.0005% to 10%, more preferably from 0.001% to 5%, more preferably from 0.005% to 2.5%, most preferably from 0.01% to 1% of one or more molecules selected from the group consisting of: 6-(R,S)-ethyl-6,9-dimethyldec-8-en-5-ol and stereoisomers thereof; and mixtures thereof; and an adjunct ingredient.

4. A consumer product according to claim 3, said consumer product being a cleaning and/or treatment composition, said composition comprising, based on total composition weight, from 0.0001% to 25%, preferably from 0.0005% to 10%, more preferably from 0.001% to 5%, more preferably from 0.005% to 2.5%, most preferably from 0.01% to 1% of one or more molecules selected from the group consisting of 6-(R,S)-ethyl-6,9-dimethyldec-8-en-5-ol.

5. A consumer product according to any one of claims 3 to 4, said consumer product being a fabric and/or hard surface cleaning and/or treatment composition, said composition comprising, based on total composition weight, from 0.00001% to 25%, preferably from 0.00005% to 10%, more preferably from 0.0001% to 5%, more preferably from 0.0005% to 1.0%, most preferably from 0.001% to 0.5% of one or more molecules selected from the group consisting of 6-(R,S)-ethyl-6,9-dimethyldec-8-en-5-ol.

6. A consumer product according to claim 3, said consumer product being a detergent, said detergent comprising, based on total detergent weight, from 0.00001% to 25%, preferably from 0.00005% to 10%, more preferably from 0.0001% to 5%, more preferably from 0.0005% to 1.0%, most preferably from 0.001% to 0.5% of one or more molecules selected from the group consisting of 6-(R,S)-ethyl-6,9-dimethyldec-8-en-5-ol.

7. A consumer product according to claim 3, said consumer product being a highly compacted consumer product, said highly compacted consumer product comprising, based on total highly compacted consumer product weight, from 0.00001% to 25%, preferably from 0.00005% to 10%, more preferably from 0.0001% to 5%, more preferably from 0.0005% to 1.0%, most preferably from 0.001% to 0.5% of one or more molecules selected from the group consisting of 6-(R,S)-ethyl-6,9-dimethyldec-8-en-5-ol.

8. A perfume delivery system comprising from 0.001% to 50%, preferably from 0.005% to 30%, more preferably from 0.01% to 10%, more preferably from 0.025% to 5%, most preferably from 0.025% to 1% of one or more molecules selected from the group consisting of 6-(R,S)-ethyl-6,9-dimethyldec-8-en-5-ol; wherein said perfume delivery system is selected from a polymer assisted delivery system; a molecule-assisted delivery system; a fiber-assisted delivery system; an amine assisted delivery system; a cyclodextrin delivery system; a starch encapsulated accord; and/or an inorganic carrier delivery system; or a pro-perfume
(i) preferably when said perfume delivery system is a nanocapsule or a microcapsule, said perfume delivery system comprises, based on total nanocapsule or microcapsule weight, from 0.1% to 99%, preferably from 25% to 95%, more preferably from 30 to 90%, more preferably from 45% to 90%, most preferably from 65% to 90% of one or more molecules selected from the group consisting of 6-(R,S)-ethyl-6,9-dimethyldec-8-en-5-ol;
(ii) preferably when said perfume delivery system is a starch encapsulated accord, said perfume delivery system comprises, based on total starch encapsulate or starch agglomerate weight, from 0.1% to 99%, preferably from 25% to 95%, more preferably from 30 to 90%, more preferably from 45% to 90%, most preferably from 65% to 90% of one or more molecules selected from the group consisting of 6-(R,S)-ethyl-6,9-dimethyldec-8-en-5-ol;
(iii) preferably when said perfume delivery system is a cyclodextrin delivery system, said perfume delivery system comprises, based on total cyclodextrin delivery system weight, from 0.1% to 99%, preferably from 2.5% to 75%, more preferably from 5% to 60%, more preferably from 5% to 50%, most preferably from 5% to 25% of one or more molecules selected from the group consisting of 6-(R,S)-ethyl-6,9-dimethyldec-8-en-5-ol;
(iv) preferably when said perfume delivery system is a polymer assisted delivery matrix system, said perfume delivery system comprises, based on total polymer assisted delivery matrix system weight, from 0.1% to 99%, preferably from 2.5% to 75%, more preferably from 5% to 60%, more preferably from 5% to 50%, most preferably from 5% to 25% of one or more molecules selected from the group consisting of 6-(R,S)-ethyl-6,9-dimethyldec-8-en-5-ol;
(v) preferably when said perfume delivery system is an amine assisted delivery system , said perfume delivery system comprises, based on total amine assisted delivery system weight, from 1% to 99%, preferably from 2.5% to 75%, more preferably from 5% to 60%, more preferably from 5% to 50%, most preferably from 5% to 25% of one or more molecules selected from the group consisting of6-(R,S)-ethyl-6,9-dimethyldec-8-en-5-ol;
(vi) preferably when said perfume delivery system is a pro-perfume amine reaction product, said perfume delivery system comprises, based on total pro-perfume amine reaction product weight, from 0.1% to 99%, preferably from 1% to 99%, more preferably from 5% to 90%, more preferably from 10% to 75%, more preferably from 20% to 75%, most preferably from 25% to 60% of one or more molecules selected from the group consisting of 6-(R,S)-ethyl-6,9-dimethyldec-8-en-5-ol.

9. A consumer product comprising, based on total consumer product weight, from 0.001% to 20%, preferably from 0.01% to 10%, more preferably from 0.05% to 5%, most preferably from 0.1% to 0.5% of a perfume delivery system selected from the perfume delivery systems of claim 8 and mixtures thereof.

## Patentansprüche

1. Molekül, ausgewählt aus der Gruppe bestehend aus: 6-(R,S)-Ethyl-6,9-dimethyldec-8-en-5-ol und Stereoisomere davon.

2. Duftstoff oder sinnlich wahrgenommene Substanz, bestehend, bezogen auf das Gesamtgewicht des Duftstoffs, von zu 0,01 % bis 50 %, vorzugsweise von zu 0,1 % bis 15 %, mehr bevorzugt von zu 0,1 % bis 10 %, am meisten bevorzugt von zu 0,5 % bis 10 % aus einem oder mehreren Molekülen, ausgewählt aus der Gruppe bestehend aus: 6-(R,S)-Ethyl-6,9-dimethyldec-8-en-5-ol und Stereoisomere davon; und Mischungen davon; und ein optionales Lösungsmittel.

3. Endprodukt, bestehend, bezogen auf das Gesamtgewicht des Endprodukts, von zu 0,0001 % bis 25 %, vorzugsweise von zu 0,0005 % bis 10 %, mehr bevorzugt von zu 0,001 % bis 5 %, mehr bevorzugt von zu 0,005 % bis 2,5 %, am meisten bevorzugt von zu 0,01 % bis 1 % aus einem oder mehreren Molekülen, ausgewählt aus der Gruppe bestehend aus: 6-(R,S)-Ethyl-6,9-dimethyldec-8-en-5-ol und Stereoisomere davon; und Mischungen davon; und einen Zusatzbestandteil.

4. Endprodukt nach Anspruch 3, wobei das Endprodukt eine Reinigungs- und/oder Behandlungszusammensetzung ist, die Zusammensetzung, bestehend, bezogen auf das Gesamtgewicht der Zusammensetzung, von zu 0,0001 % bis 25 %, vorzugsweise von zu 0,0005 % bis 10 %, mehr bevorzugt von zu 0,001 % bis 5 %, mehr bevorzugt von zu 0,005 % bis 2,5 %, am meisten bevorzugt von zu 0,01 % bis 1 % aus einem oder mehreren Moleküle, ausgewählt aus der Gruppe bestehend aus 6-(R,S)-Ethyl-6,9-dimethyldec-8-en-5-ol.

5. Endprodukt nach einem der Ansprüche 3 bis 4, wobei das Endprodukt eine Reinigungs- und/oder Behandlungszusammensetzung für Stoff und/oder harte Oberflächen ist, die Zusammensetzung, bestehend, bezogen auf das Gesamtgewicht der Zusammensetzung, von zu 0,00001 % bis 25 %, vorzugsweise von zu 0,00005 % bis 10 %, mehr bevorzugt von zu 0,0001 % bis 5 %, mehr bevorzugt von zu 0,0005 % bis 1,0 %, am meisten bevorzugt von zu 0,001 % bis 0,5 % aus einem oder mehreren Molekülen, ausgewählt aus der Gruppe bestehend aus 6-(R,S)-Ethyl-6,9-dimethyldec-8-en-5-ol.

6. Endprodukt nach Anspruch 3, wobei das Endprodukt ein Waschmittel ist, das Waschmittel, bestehend, bezogen auf das Gesamtgewicht des Waschmittels, von zu 0,00001 % bis 25 %, vorzugsweise von zu 0,00005 % bis 10 %, mehr bevorzugt von zu 0,0001 % bis 5 %, mehr bevorzugt von zu 0,0005 % bis 1,0 %, am meisten bevorzugt von zu 0,001 % bis 0,5 % aus einem oder mehreren Molekülen, ausgewählt aus der Gruppe bestehend aus 6-(R,S)-Ethyl-6,9-dimethyldec-8-en-5-ol.

7. Endprodukt nach Anspruch 3, wobei das Endprodukt ein hoch verdichtetes Endprodukt ist, das hoch verdichtete Endprodukt, bestehend, bezogen auf das Gesamtgewicht des hoch verdichteten Endprodukts, von zu 0,00001 % bis 25 %, vorzugsweise von zu 0,00005 % bis 10 %, mehr bevorzugt von zu 0,0001 % bis 5 %, mehr bevorzugt von zu 0,0005 % bis 1,0 %, am meisten bevorzugt von zu 0,001 % bis 0,5 % aus einem oder mehreren Molekülen, ausgewählt aus der Gruppe bestehend aus 6-(R,S)-Ethyl-6,9-dimethyldec-8-en-5-ol.

8. Duftstoffabgabesystem, bestehend von zu 0,001 % bis 50 %, vorzugsweise von zu 0,005 % bis 30 %, mehr bevorzugt von zu 0,01 % bis 10 %, mehr bevorzugt von zu 0,025 % bis 5 %, am meisten bevorzugt von zu 0,025 % bis 1 % aus einem oder mehreren Molekülen, ausgewählt aus der Gruppe bestehend aus 6-(R,S)-Ethyl-6,9-dimethyldec-8-en-5-ol; wobei das Duftstoffabgabesystem ausgewählt ist aus einem polymerunterstützten Abgabesystem; einem molekülunterstützten Abgabesystem; einem faserunterstützten Abgabesystem; einem aminunterstützten Abgabesystem; einem Cyclodextrinabgabesystem; einer in Stärke eingekapselten Duftnote; und/oder einem anorganischen Trägerabgabesystem; oder einem Pro-Duftstoff
(i) vorzugsweise wenn das Duftstoffabgabesystem eine Nanokapsel oder eine Mikrokapsel ist, das Duftstoffabgabesystem, bezogen auf das Gesamtgewicht der Nanokapsel oder der Mikrokapsel, von zu 0,1 % bis 99 %, vorzugsweise von zu 25 % bis 95 %, mehr bevorzugt von zu 30 bis 90 %, mehr bevorzugt von zu 45 % bis 90 %, am meisten bevorzugt von zu 65 % bis 90 %, aus einem oder mehreren Molekülen, ausgewählt aus der Gruppe bestehend aus 6-(R,S)-Ethyl-6,9-dimethyldec-8-en-5-ol, besteht;
(ii) vorzugsweise wenn das Duftstoffabgabesystem eine mit Stärke eingekapselte Duftnote ist, das Duftstoffabgabesystem, bezogen auf das Gesamtgewicht der eingekapselten Gesamtstärke oder dem Stärke-Agglomerat, von zu 0,1 % bis 99 %, vorzugsweise von zu 25 % bis 95 %, mehr bevorzugt von zu 30 bis 90 %, mehr bevorzugt von zu 45 % bis 90 %, am meisten bevorzugt von zu 65 % bis 90 % aus einem oder mehreren Molekülen, ausgewählt aus der Gruppe bestehend aus 6-(R,S)-Ethyl-6,9-dimethyldec-8-en-5-ol, besteht;
(iii) vorzugsweise wenn das Duftstoffabgabesystem ein Cyclodextrinabgabesystem ist, das Duftstoffabgabesystem, bezogen auf das Gesamtgewicht des Cyclodextrinabgabesystems, von zu 0,1 % bis 99 %, vorzugsweise von zu 2,5 % bis 75 %, mehr bevorzugt von zu 5 % bis 60 %, mehr bevorzugt von zu 5 % bis 50 %, am meisten bevorzugt von zu 5 % bis 25 % aus einem oder mehreren Molekülen, ausgewählt aus der Gruppe bestehend aus 6-(R,S)-Ethyl-6,9-dimethyldec-8-en-5-ol, besteht;
(iv) vorzugsweise wenn das Duftstoffabgabesystem ein polymerunterstütztes Abgabematrixsystem ist, das Duftstoffabgabesystem, bezogen auf das Gesamtgewicht des polymerunterstützten Abgabematrixsystems, von zu 0,1 % bis 99 %, vorzugsweise von zu 2,5 % bis 75 %, mehr bevorzugt von zu 5 % bis 60 %, mehr bevorzugt von zu 5 % bis 50 %, am meisten bevorzugt von zu 5 % bis 25 % aus einem oder mehreren Molekülen, ausgewählt aus der Gruppe bestehend aus 6-(R,S)-Ethyl-6,9-dimethyldec-8-en-5-ol, besteht;
(v) vorzugsweise wenn das Duftstoffabgabesystem ein aminunterstütztes Abgabesystem ist, das Duftstoffabgabesystem, bezogen auf das Gesamtgewicht des aminunterstützten Abgabesystems, von zu 1 % bis 99 %, vorzugsweise von zu 2,5 % bis 75 %, mehr bevorzugt von zu 5 % bis 60 %, mehr bevorzugt von zu 5 % bis 50 %, am meisten bevorzugt von zu 5 % bis 25 % aus einem oder mehreren Molekülen, ausgewählt aus der Gruppe bestehend aus 6-(R,S)-Ethyl-6,9-dimethyldec-8-en-5-ol, besteht;
(vi) vorzugsweise wenn das Duftstoffabgabesystem ein Pro-Duftstoffamin-Reaktionsprodukt ist, das Duftstoffabgabesystem, bezogen auf das Gesamtgewicht des Pro-Duftstoffamin-Reaktionsprodukts, von zu 0,1 % bis 99 %, vorzugsweise von zu 1 % bis 99 %, mehr bevorzugt von zu 5 % bis 90 %, mehr bevorzugt von zu 10 % bis 75 %, mehr bevorzugt von zu 20 % bis 75 %, am meisten bevorzugt von zu 25 % bis 60 %, aus einem oder mehreren Molekülen, ausgewählt aus der Gruppe bestehend aus 6-(R,S)-Ethyl-6,9-dimethyldec-8-en-5-ol, besteht.

9. Endprodukt bestehend, bezogen auf Gesamtgewicht des Endprodukts von zu 0,001 % bis 20 %, vorzugsweise von zu 0,01 % bis 10 %, mehr bevorzugt von zu 0,05 % bis 5 %, am meisten bevorzugt von zu 0,1 % bis 0,5 % aus einem Parfüm-Abgabesystem, ausgewählt aus den Parfüm-Abgabesystemen nach Anspruch 8 und Mischungen davon.

## Revendications

1. Molécule choisie dans le groupe constitué de : 6-(R,S)-éthyl-6,9-diméthyldéc-8-én-5-ol et stéréo-isomères de celui-ci.

2. Composition de parfum ou d'agent sensoriel comprenant, sur la base du poids total de parfum, de 0,01 % à 50 %, de préférence de 0,1 % à 15 %, plus préférablement de 0,1 % à 10 %, le plus préférablement de 0,5 % à 10 % d'une ou plusieurs molécules choisies dans le groupe constitué de : 6-(R,S)-éthyl-6,9-diméthyldéc-8-én-5-ol et stéréo-isomères de celui-ci ; et mélanges de ceux-ci ; et un solvant facultatif.

3. Produit de consommation comprenant, sur la base du poids total de produit de consommation, de 0,0001 % à 25 %, de préférence de 0,0005 % à 10 %, plus préférablement de 0,001 % à 5 %, plus préférablement de 0,005 % à 2,5 %, le plus préférablement de 0,01 % à 1 % d'une ou plusieurs molécules choisies dans le groupe constitué de : 6-(R,S)-éthyl-6,9-diméthyldéc-8-én-5-ol et stéréo-isomères de celui-ci ; et mélanges de ceux-ci ; et un ingrédient additionnel.

4. Produit de consommation selon la revendication 3, ledit produit de consommation étant une composition de nettoyage et/ou de traitement, ladite composition comprenant, sur la base du poids total de composition, de 0,0001 % à 25 %, de préférence de 0,0005 % à 10 %, plus préférablement de 0,001 % à 5 %, plus préférablement de 0,005 % à 2,5 %, le plus préférablement de 0,01 % à 1 % d'une ou plusieurs molécules choisies dans le groupe constitué de 6-(R,S)-éthyl-6,9-diméthyldéc-8-én-5-ol.

5. Produit de consommation selon l'une quelconque des revendications 3 à 4, ledit produit de consommation étant une composition de nettoyage et/ou de traitement de tissu et/ou de surface dure, ladite composition comprenant, sur la base du poids total de composition, de 0,00001 % à 25 %, de préférence de 0,00005 % à 10 %, plus préférablement de 0,0001 % à 5 %, plus préférablement de 0,0005 % à 1,0 %, le plus préférablement de 0,001 % à 0,5 % d'une ou plusieurs molécules choisies dans le groupe constitué de 6-(R,S)-éthyl-6,9-diméthyldéc-8-én-5-ol.

6. Produit de consommation selon la revendication 3, ledit produit de consommation étant un détergent, ledit détergent comprenant, sur la base du poids total de détergent, de 0,00001 % à 25 %, de préférence de 0,00005 % à 10 %, plus préférablement de 0,0001 % à 5 %, plus préférablement de 0,0005 % à 1,0 %, le plus préférablement de 0,001 % à 0,5 % d'une ou plusieurs molécules choisies dans le groupe constitué de 6-(R,S)-éthyl-6,9-diméthyldéc-8-én-5-ol.

7. Produit de consommation selon la revendication 3, ledit produit de consommation étant un produit de consommation fortement compacté, ledit produit de consommation fortement compacté comprenant, sur la base du poids total de produit de consommation fortement compacté, de 0,00001 % à 25 %, de préférence de 0,00005 % à 10 %, plus préférablement de 0,0001 % à 5 %, plus préférablement de 0,0005 % à 1,0 %, le plus préférablement de 0,001 % à 0.5 % d'une ou plusieurs molécules choisies dans le groupe constitué de 6-(R,S)-éthyl-6,9-diméthyldéc-8-én-5-ol.

8. Système de libération de parfum comprenant de 0,001 % à 50 %, de préférence de 0,005 % à 30 %, plus préférablement de 0,01 % à 10 %, plus préférablement de 0,025 % à 5 %, le plus préférablement de 0,025 % à 1 % d'une ou plusieurs molécules choisies dans le groupe constitué de 6-(R,S)-éthyl-6,9-diméthyldéc-8-én-5-ol ; dans lequel ledit système de libération de parfum est choisi parmi un système de libération assistée par polymère ; un système de libération assistée par molécule ; un système de libération assistée par fibre ; un système de libération assistée par amine ; un système de libération par cyclodextrine ; un accord encapsulé dans de l'amidon ; et/ou un système de libération par véhicule inorganique ; ou un proparfum
(i) de préférence, lorsque ledit système de libération de parfum est une nanogélule ou une microgélule, ledit système de libération de parfum comprend, sur la base du poids total de nanogélule ou microgélule, de 0,1 % à 99 %, de préférence de 25 % à 95 %, plus préférablement de 30 à 90 %, plus préférablement de 45 % à 90 %, le plus préférablement de 65 % à 90 % d'une ou plusieurs molécules choisies dans le groupe constitué de 6-(R,S)-éthyl-6,9-diméthyldéc-8-én-5-ol ;
(ii) de préférence, lorsque ledit système de libération de parfum est un accord encapsulé dans de l'amidon, ledit système de libération de parfum comprend, sur la base du poids total d'encapsulat d'amidon ou d'agglomérat d'amidon, de 0,1 % à 99 %, de préférence de 25 % à 95 %, plus préférablement de 30 à 90 %, plus préférablement de 45 % à 90 %, le plus préférablement de 65 % à 90 % d'une ou plusieurs molécules choisies dans le groupe constitué de 6-(R,S)-éthyl-6,9-diméthyldéc-8-én-5-ol ;
(iii) de préférence, lorsque ledit système de libération de parfum est un système de libération par cyclodextrine, ledit système de libération de parfum comprend, sur la base du poids total de système de libération par cyclodextrine, de 0,1 % à 99 %, de préférence de 2,5 % à 75 %, plus préférablement de 5 % à 60 %, plus préférablement de 5 % à 50 %, le plus préférablement de 5 % à 25 % d'une ou plusieurs molécules choisies dans le groupe constitué de 6-(R,S)-éthyl-6,9-diméthyldéc-8-én-5-ol ;
(iv) de préférence, lorsque ledit système de libération de parfum est un système de matrice de libération assistée par polymère, ledit système de libération de parfum comprend, sur la base du poids total de système de matrice de libération assistée par polymère, de 0,1 % à 99 %, de préférence de 2,5 % à 75 %, plus préférablement de 5 % à 60 %, plus préférablement de 5 % à 50 %, le plus préférablement de 5 % à 25 % d'une ou plusieurs molécules choisies dans le groupe constitué de 6-(R,S)-éthyl-6,9-diméthyldéc-8-én-5-ol ;
(v) de préférence, lorsque ledit système de libération de parfum est un système de libération assistée par amine, ledit système de libération de parfum comprend, sur la base du poids total de système de libération assistée par amine, de 1 % à 99 %, de préférence de 2,5 % à 75 %, plus préférablement de 5 % à 60 %, plus préférablement de 5 % à 50 %, le plus préférablement de 5 % à 25 % d'une ou plusieurs molécules choisies dans le groupe constitué de 6-(R,S)-éthyl-6,9-diméthyldéc-8-én-5-ol ;
(vi) de préférence, lorsque ledit système de libération de parfum est un produit de réaction d'amine à proparfum, ledit système de libération de parfum comprend, sur la base du poids total de produit de réaction d'amine à proparfum, de 0,1 % à 99 %, de préférence de 1 % à 99 %, plus préférablement de 5 % à 90 %, plus préférablement de 10 % à 75 %, plus préférablement de 20 % à 75 %, le plus préférablement de 25 % à 60 % d'une ou plusieurs molécules choisies dans le groupe constitué de 6-(R,S)-éthyl-6,9-diméthyldéc-8-én-5-ol.

9. Produit de consommation comprenant, sur la base du poids total de produit de consommation, de 0,001 % à 20 %, de préférence de 0,01 % à 10 %, plus préférablement de 0,05 % à 5 %, le plus préférablement de 0,1 % à 0,5 % d'un système de libération de parfum choisi parmi les systèmes de libération de parfum selon la revendication 8 et des mélanges de ceux-ci.
